# EUROPEAN PATENT APPLICATION

(11) **EP 4 585 691 A1**
(43) Date of publication of application: **16.07.2025**
(21) Application number: 23863512.2
(22) Date of filing: 06.09.2023
(51) Int. Cl.: C12N 15/63, C12N 15/113

(54) **SELF-CIRCULARIZED RNA STRUCTURE**

(30) Priority: 06.09.2022 KR 20220112826
(71) Applicant: RZNOMICS INC., Yongin-si, Gyeonggi-do 16890 (KR)
(72) Inventor: LEE, Seong Wook, Seoul 02780 (KR); LEE, Kyung Hyun, Seongnam-si, Gyeonggi-do 13624 (KR); KIM, Seong Cheol, Seongnam-si, Gyeonggi-do 13505 (KR)
(74) Representative: De Clercq & Partners
(86) International application number: PCT/KR2023/013376
(87) International publication number: WO 2024/054048

(57) **Abstract**

A self-circularized RNA structure of the present invention can be expressed in a DNA vector and, at the same time, form circRNA by being circularized through a self-targeting and splicing reaction, wherein the circRNA consists of only a gene of interest. The gene of interest includes an IRES region, an initiation codon and a termination codon, and thus enables the rapid expression of a peptide and a protein.

## Description

### TECHNICAL FIELD

The following description relates to RNA constructs with improved self-circularization efficiency.

The present disclosure stems from a project of the National Research Foundation of Korea named "Core Technology Development for Next Generation Vaccines for Infectious Diseases" (Project No. 2022M3E5F1017657) and was supported with funding by the Ministry of Science and ICT.

### BACKGROUND ART

Circular RNAs (circRNAs, cRNAs) are single-stranded transcripts covalently linked. Through RNA-seq data and a newly developed bioinformatics approach, more than tens of thousands of circRNA types have been identified in various living organisms. In eukaryotes, it is known that a circRNA may be generated through back-splicing from mRNA and may regulate gene expression by performing a microRNA sponge function in vivo. It is not known whether a circRNA induces immunogenicity, but it exists very stably in vivo due to its structural characteristics.

Recently, the development of therapeutic agents using messenger RNA (mRNA) has been active. However, mRNA has a limitation in that it easily degrades in vivo and has a relatively short half-life. In order to overcome this limitation, studies on attaching poly(A) tail to mRNA to improve stability are in progress. In the same vein, US 10,953,033 discloses a circRNA for the purpose of gene expression in vivo based on the structural characteristics of the circRNA.

### DISCLOSURE OF THE INVENTION

### TECHNICAL GOALS

A technical task of the present disclosure is to provide an RNA construct that is circularized by performing a self-targeting and splicing reaction.

However, the technical task of the present disclosure is not limited to that described above, and other tasks not mentioned herein will be clearly understood by one of ordinary skill in the art from the following description.

### TECHNICAL SOLUTIONS

According to an aspect of the present disclosure, there is provided a self-circularized RNA construct having the following structure:
5' - IGS (internal guide sequence) - ribozyme - gene of interest - target site - 3'.

In one embodiment of the present disclosure, the IGS region and the target site form a guanine (G) : uracil (U) wobble base pair, in which the guanine forming the wobble base pair may be located at the 5' end of the IGS region, the uracil forming the wobble base pair may be located at the 3' end of a target site region, and the IGS region may consist of adenine (A) or uracil in addition to the bases forming the wobble base pair.

In another embodiment of the present disclosure, the IGS region may include or consist of a nucleotide sequence represented by 5'-GNNNNN-3', and the target site region may include or consist of a nucleotide sequence represented by 5'-N'N'N'N'N'U-3'. N of the IGS region and N' of the target site region may each independently be A or U, wherein the IGS region may include A and U in a ratio of 2:3 or 3:2.

In yet another embodiment of the present disclosure, a nucleotide sequence of the IGS region may be reverse complementary to a nucleotide sequence of the target site region except for the guanine.

In another embodiment of the present disclosure, the ribozyme may be a Group I intron ribozyme, and the ribozyme may include or consist of a nucleotide sequence represented by SEQ ID NO: 6.

In yet another embodiment of the present disclosure, the construct may include a nucleotide extending in a 5' direction of the IGS region such that a P1 helix and a P10 helix may be formed. In this context, the P1 helix may be formed in a region in which the complementary binding between the IGS region and the target site is formed with a nucleotide extending in a 3' direction of the target site, and the P10 helix may be formed in a region in which complementary binding to a sequence reverse complementary to the extended nucleotide located between a ribozyme and a GOI region is formed with the nucleotide extending in the 5' direction of the IGS region. The length of the extended nucleotide forming the P1 helix may be 3-nt, and the length of the extended nucleotide forming the P10 helix may be 6-nt.

In yet another embodiment of the present disclosure, the construct may form a P1 helix but not a P10 helix.

In yet another embodiment of the present disclosure, the construct may include an antisense sequence (AS) and an antisense binding sequence (ABS) region capable of complementary binding to each other at the 5' end and the 3' end.

In yet another embodiment of the present disclosure, the ABS region may consist of a sequence reverse complementary to the AS region.

In yet another embodiment of the present disclosure, the length of the AS region may vary depending on GOI, but may be 10-nt to 500-nt, preferably more than 50-nt and less than 400-nt, and more preferably 150-nt to 350-nt.

In yet another embodiment of the present disclosure, the GOI region may include an internal ribosome entry site (IRES) region at the 5' end, and may include an initiation codon and a termination codon.

In yet another embodiment of the present disclosure, the construct may further include a spacer region including a random nucleotide sequence, in which the spacer region may be located between the IGS region and the gene of interest region and/or between the gene of interest region and the target site region.

In yet another embodiment of the present disclosure, the spacer region may include or consist of poly(A), and the poly(A) is a polynucleotide in which adenine (A) is repeatedly linked, in which the A may be linked repeatedly 10 to 50 times, and preferably linked repeatedly 30 times.

### EFFECTS

The self-circularized RNA construct of the present disclosure may be expressed in a DNA vector and simultaneously circularized through a self-targeting & splicing reaction without a separate GTP treatment to form a circRNA. The circRNA may consist only of a gene of interest, and the gene of interest has the advantage of being able to rapidly express a peptide or protein, including an IRES region, an initiation codon, and a termination codon. In addition, a circRNA has a circular structure and has a stable and long half-life because the 5' and 3' ends are not exposed. Accordingly, functional RNA such as miRNA, anti-miRNA, shRNA, aptamers, mRNA vaccines, mRNA therapeutic agents, antibodies, vaccine adjuvants, CAR-T mRNA, genomes or RNA editing guide RNA may be produced in the form of circRNA, ensuring high stability within cells.

### BRIEF DESCRIPTION OF DRAWINGS

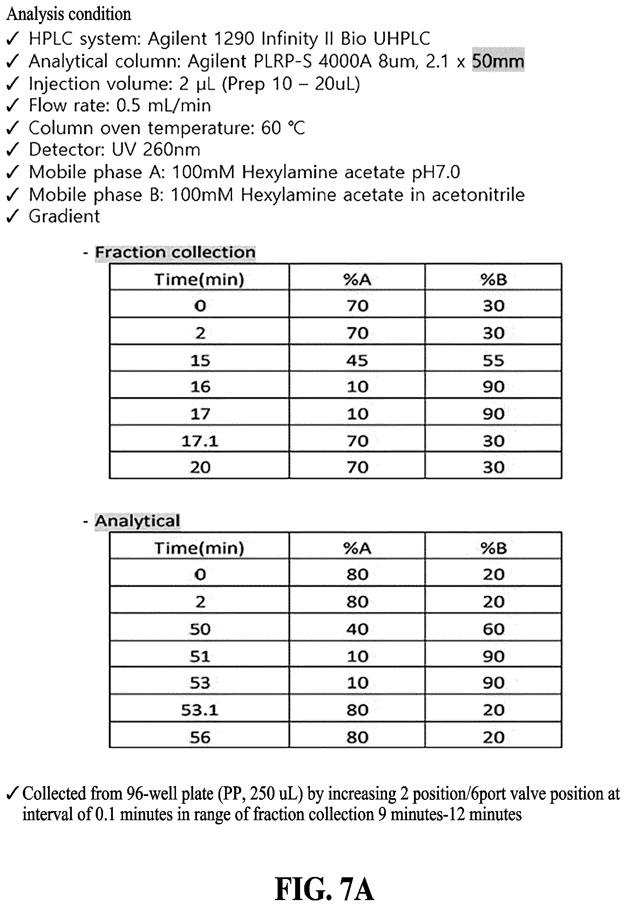

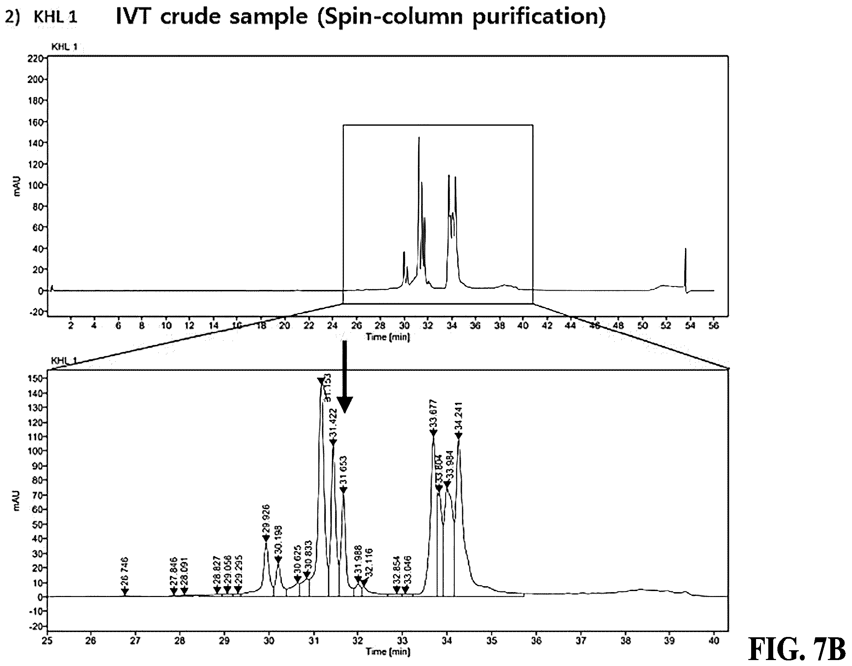

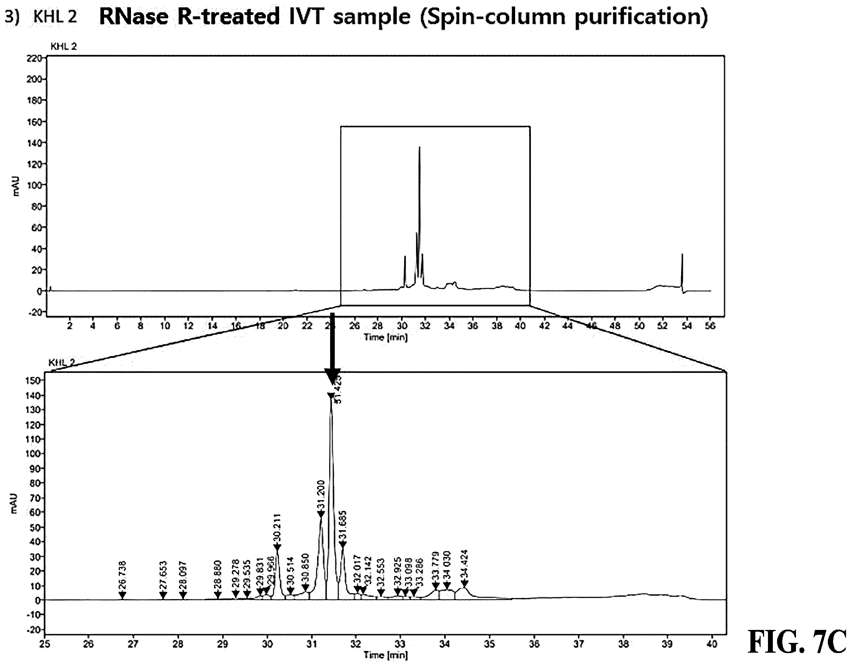

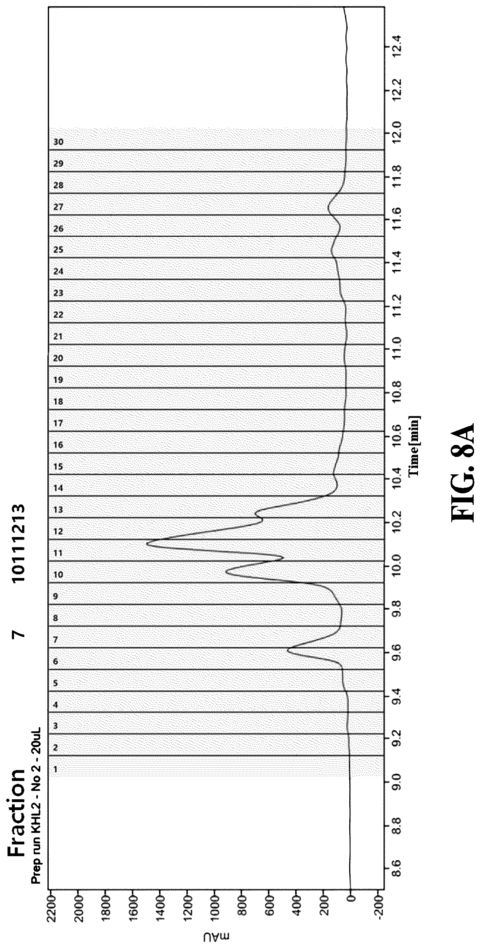

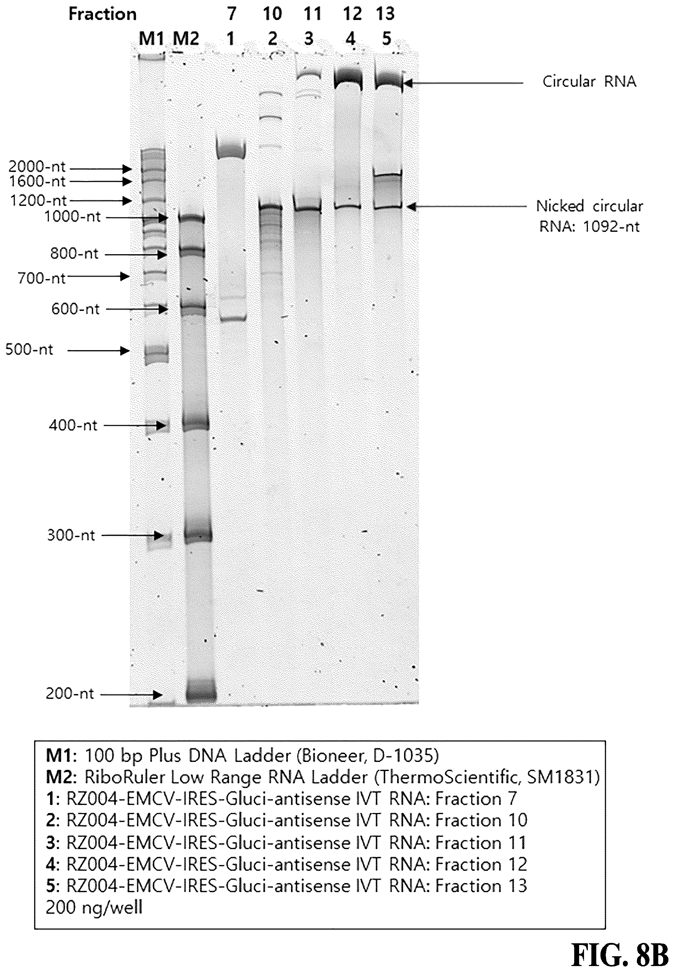

FIGS. 1A and 1B are schematic diagrams of a process in which a self-circularization RNA construct of an embodiment of the present disclosure including only IGS, ribozyme, gene of interest, and target site region is formed into a circRNA by a self-targeting and splicing (STS) reaction.
FIG. 2A is a schematic diagram of a process in which a self-circularization RNA construct of an embodiment of the present disclosure including AS, IGS, ribozyme, gene of interest, target site, and ABS region is formed into a circRNA by an STS reaction.
FIG. 2B is a schematic diagram of a self-circularization RNA construct of an embodiment of the present disclosure including IRES and a termination codon in the gene of interest region to enable transgene translation, and further including nucleotides extending in a 5' direction such that a P1 helix and P10 helix may be formed, and a process in which the construct is formed into a circRNA by an STS reaction.
FIG. 2C shows one form of a self-circularization RNA construct of an embodiment of the present disclosure.
FIG. 2D shows a DNA template nucleotide sequence for producing a self-circularization RNA construct expression vector of an embodiment of the present disclosure.

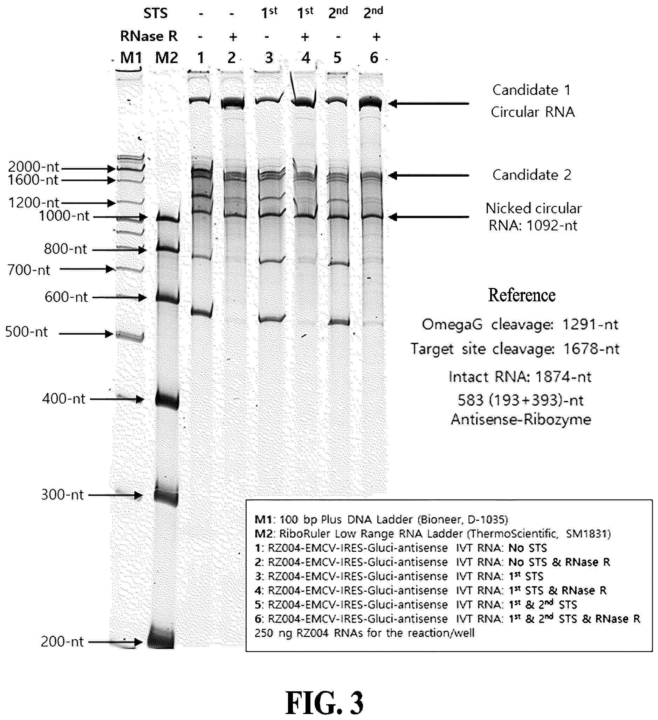

FIG. 3 shows the results of electrophoresis performed on a polyacrylamide gel to identify whether a circRNA is generated immediately after in vitro transcription of the self-circularization RNA construct expression vector of an embodiment of the present disclosure, without additional GTP treatment. Samples obtained immediately after transcription (direct STS) and samples obtained after the first and second STS reaction stages in which additional GTP treatment is performed to induce primary and secondary circularization reactions were used, and a portion of each sample was treated with RNase R to remove linear RNA, and circRNA in the samples was concentrated and electrophoresed.
FIG. 4 verifies that RNA assumed to be circRNA of the Candidate 1 band identified from the results of FIG. 3, is circRNA. FIG. 4A shows a result of extracting RNA from the Candidate 1 band and performing RT-PCR. FIG. 4B shows the sequencing result of RNA extracted from the Candidate 1 band.
FIG. 5 verifies that the RNA of the Candidate 1 band identified from the results of FIG. 4 is a monomer, and shows the results of electrophoresis after RNA assumed to be circRNA was extracted from the Candidate 1 and 2 bands identified from the results of FIG. 3 and inducing nicks by treatment with Mg²⁺.
FIG. 6 shows a result of identifying whether the self-circularization RNA construct expression vector of an embodiment of the present disclosure generates a circRNA in cells and whether the transgene included in the circRNA is expressed. Specifically, FIG. 6A shows a structure of the self-circularization RNA construct expression vector, FIG. 6B shows a result of identifying the expression of transgene through a luciferase activity assay, and FIGS. 6C and 6D show RT-PCR and sequencing results verifying that the transgene is expressed in circRNA.
FIGS. 7 and 8 show conditions for purifying circRNA by HPLC and results of purifying circRNA by HPLC.
Specifically, FIG. 7A shows HPLC analysis conditions using an Ultra HPLC system, FIG. 7B shows a result of column purification of a sample obtained immediately after in vitro transcription, and FIG. 7C shows a result of column purification after the sample was treated with RNase R. In addition, FIG. 8A shows a result of identifying a peak in the fraction obtained through column purification, and FIG. 8B shows an electrophoresis result of fractions 7, 10, 11, 12, and 13 in which peaks are prominent shown in FIG. 8A.

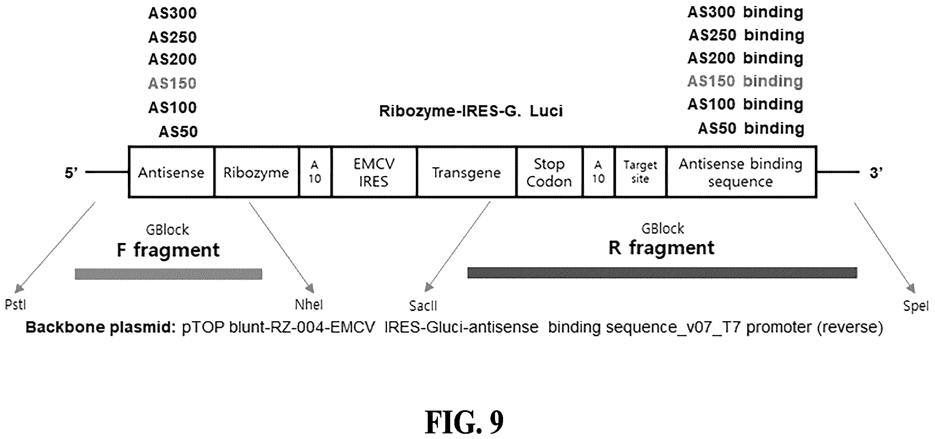

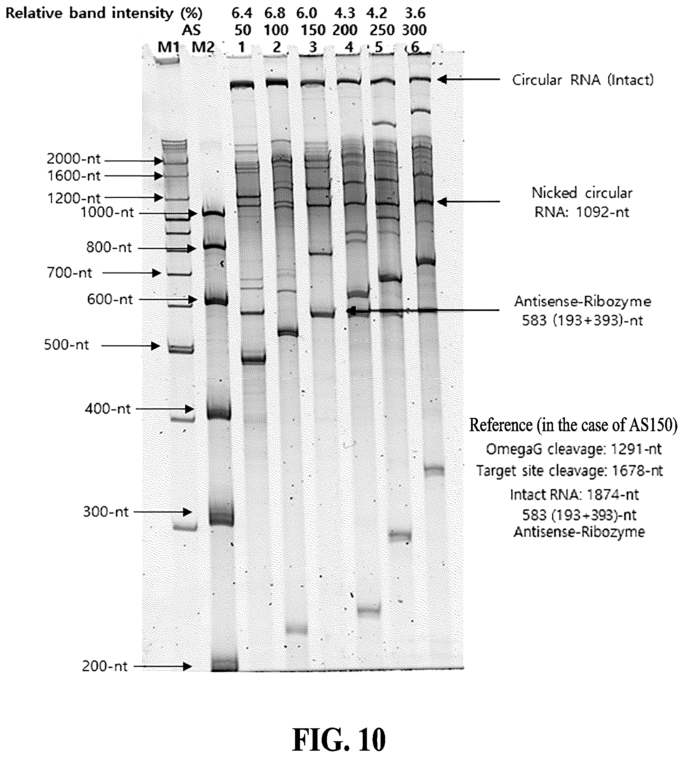

FIGS. 9 and 10 illustrate the effect of an AS region on STS reaction and circularization in a self-circularization RNA construct of an embodiment of the present disclosure. FIG. 9 shows a structure of a self-circularization RNA including AS regions of different lengths. FIG. 10 shows an electrophoresis result of a sample obtained after in vitro transcription of a vector expressing the RNA.

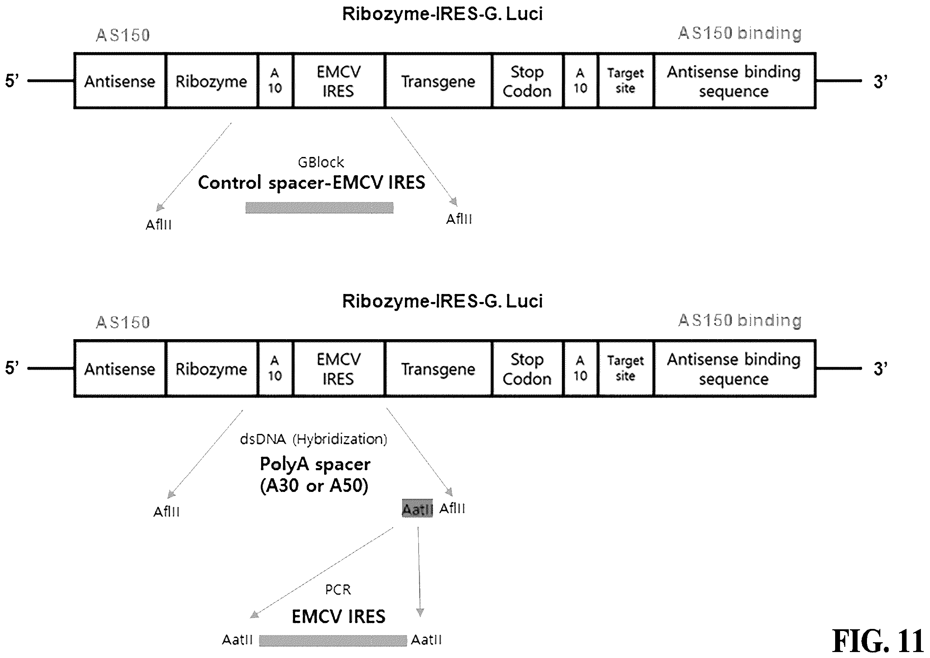

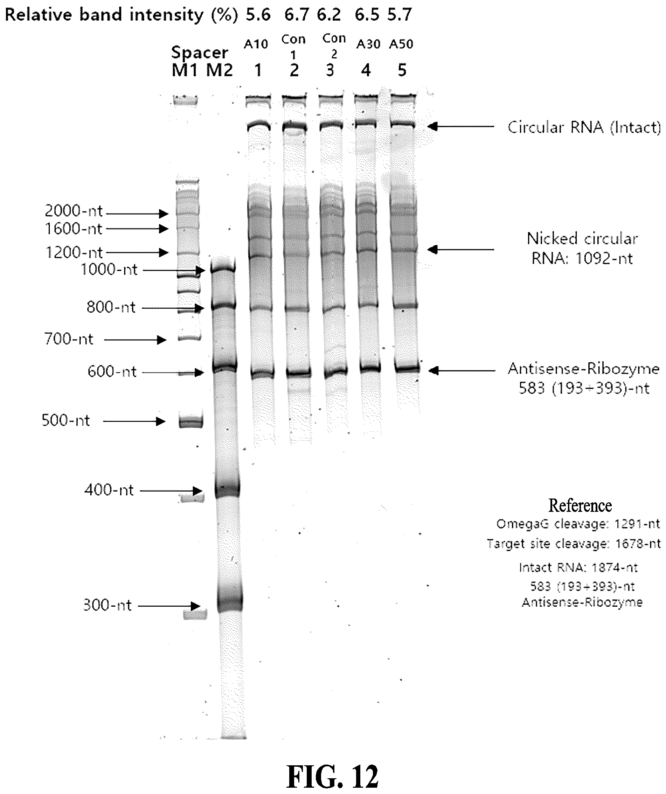

FIGS. 11 and 12 illustrate the effect of a spacer region on STS reaction and circularization in a self-circularization RNA construct of an embodiment of the present disclosure. FIG. 11 shows a structure of self-circularization RNA including a spacer of a control group (control spacer) and poly(A) spacers of various lengths. FIG. 12 shows an electrophoresis result of a sample obtained after in vitro transcription of a vector expressing the RNA.

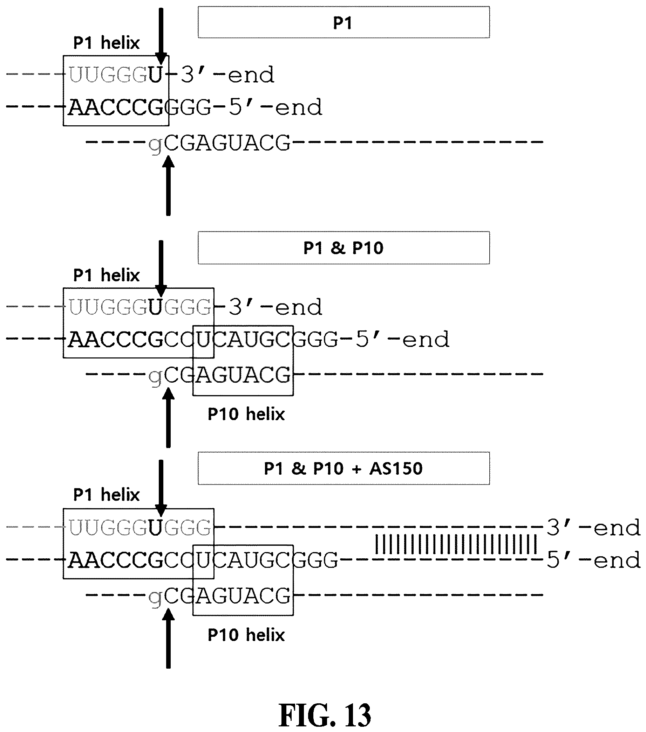

FIG. 13 indicates a nucleotide sequence near a region cleaved by a ribozyme and each region of: a self-circularization RNA including only a P1 region; self-circularization RNA including only P1 and P10 regions; and a self-circularization RNA including a P1 region, a P10 region, and an AS region.

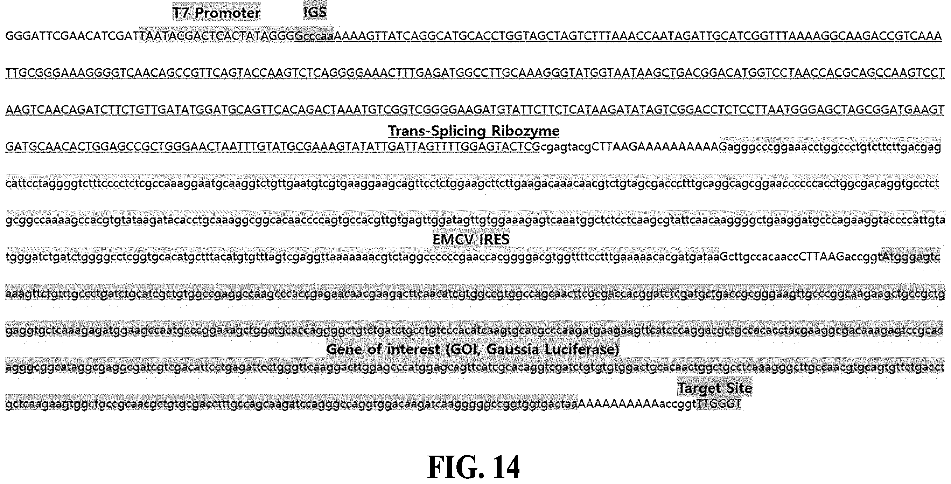

FIG. 14 shows a nucleotide sequence of a DNA template for preparing a self-circularization RNA expression vector including only a P1 region without P10 and AS regions.

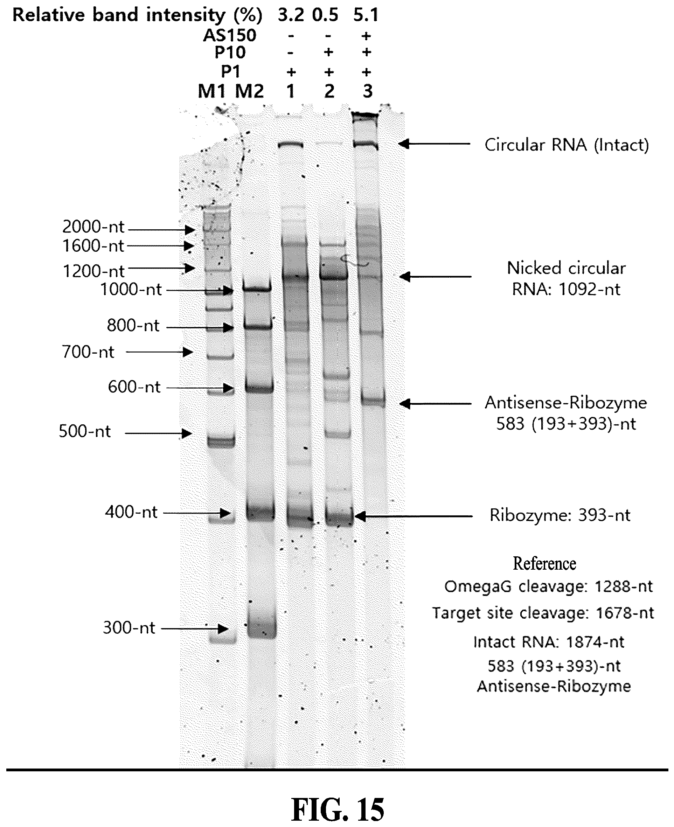

FIG. 15 shows an electrophoresis result of a sample obtained after in vitro transcription of each self-circularization RNA expression vector of FIG. 13.

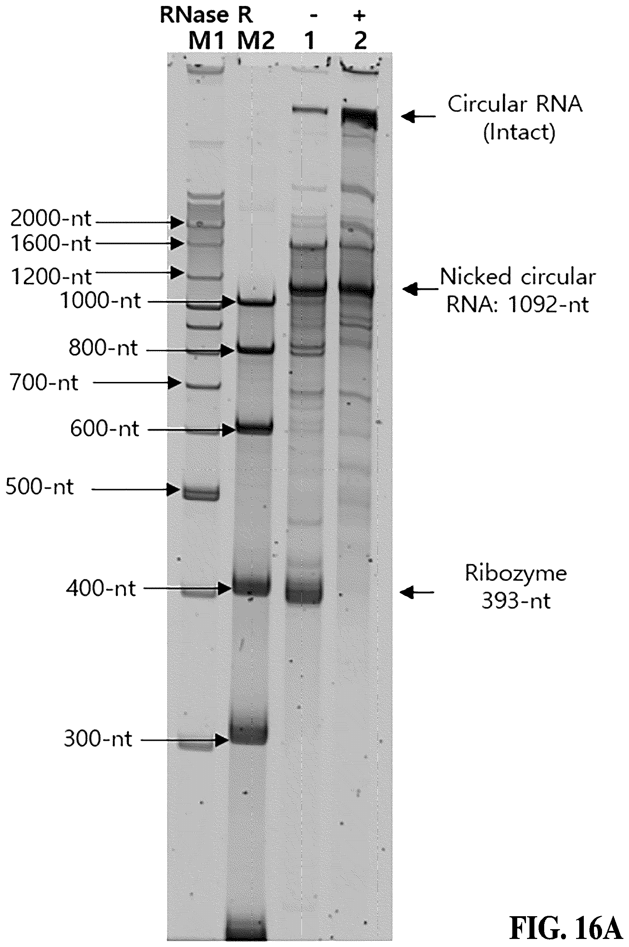

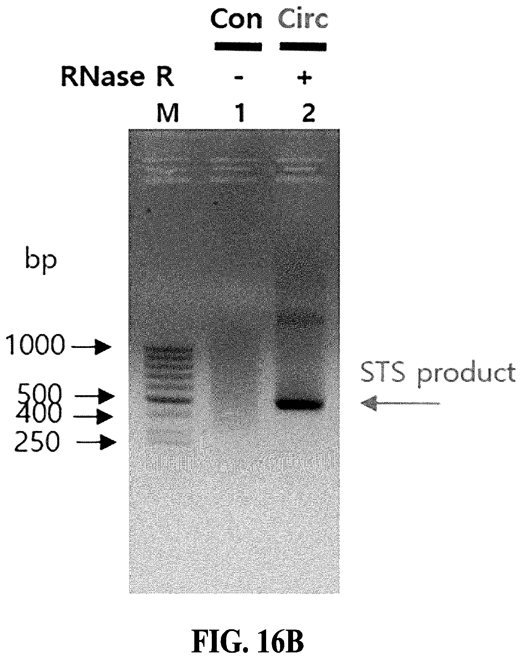

FIGS. 16A and 16B show the results of electrophoresis identifying that a self-circularization RNA construct is formed into a circRNA after in vitro transcription in a self-circularization RNA expression vector including only a P1 region without P10 and AS regions.

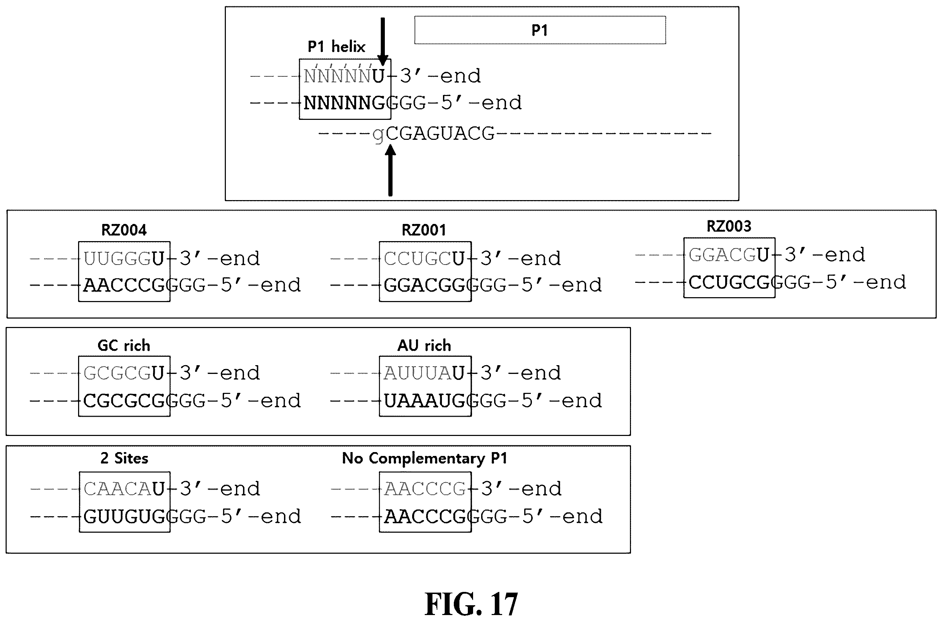

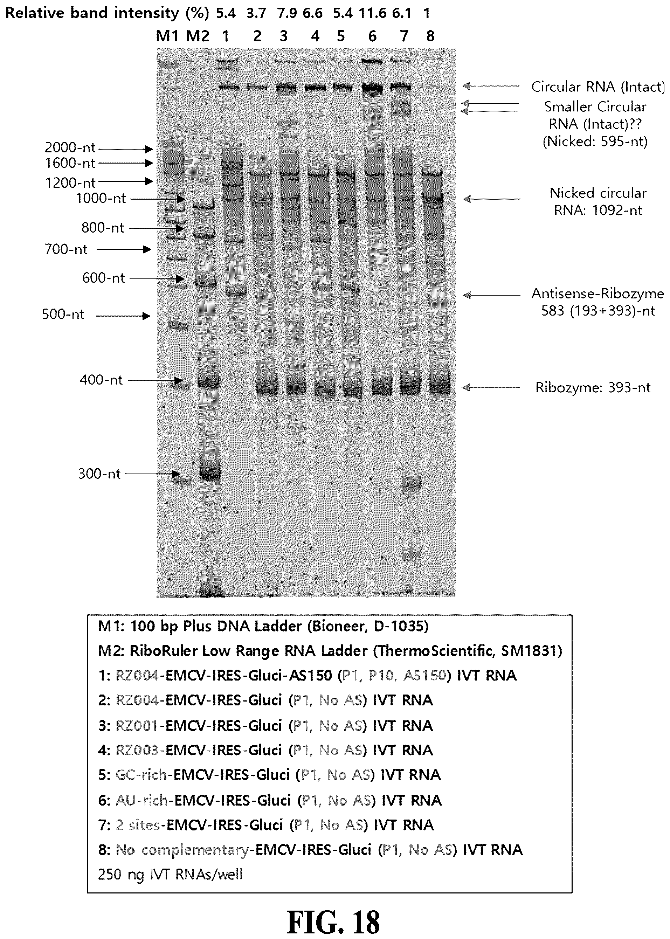

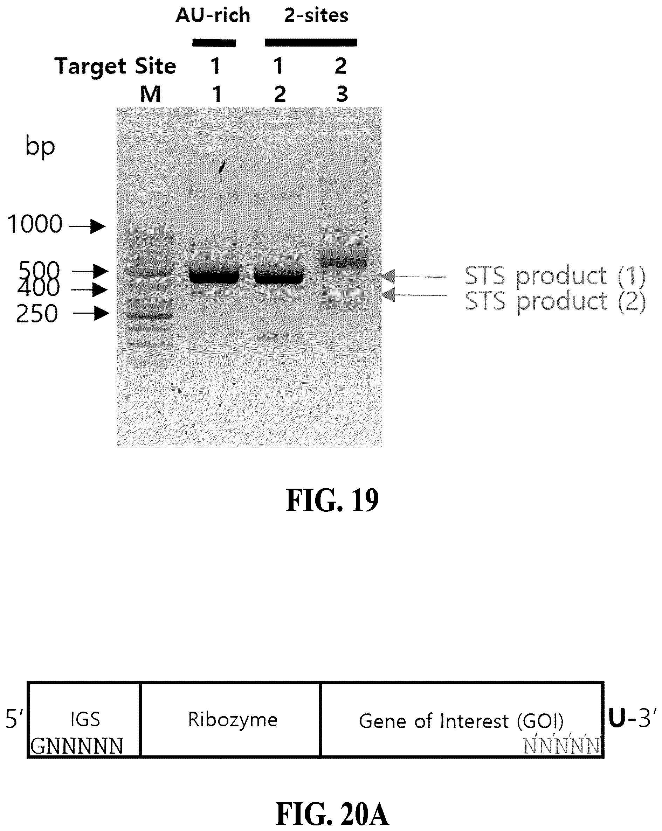

FIGS. 17 to 19 illustrate the effect of a nucleotide sequence of a P1 region on STS reaction and circularization in a self-circularization RNA construct of an embodiment of the present disclosure. Specifically, FIG. 17 shows a design of the P1 region of each different nucleotide sequence, FIG. 18 shows an electrophoresis result of a sample obtained after in vitro transcription of a self-circularization RNA expression vector having the P1 region of FIG. 17, and FIG. 19 shows an electrophoresis result of a sample obtained after in vitro transcription of a self-circularization RNA expression vector having an AU-rich P1 region and a self-circularization RNA expression vector having 2 sites for P1 formation, and shows an RT-PCR electrophoresis result identifying circular RNA.

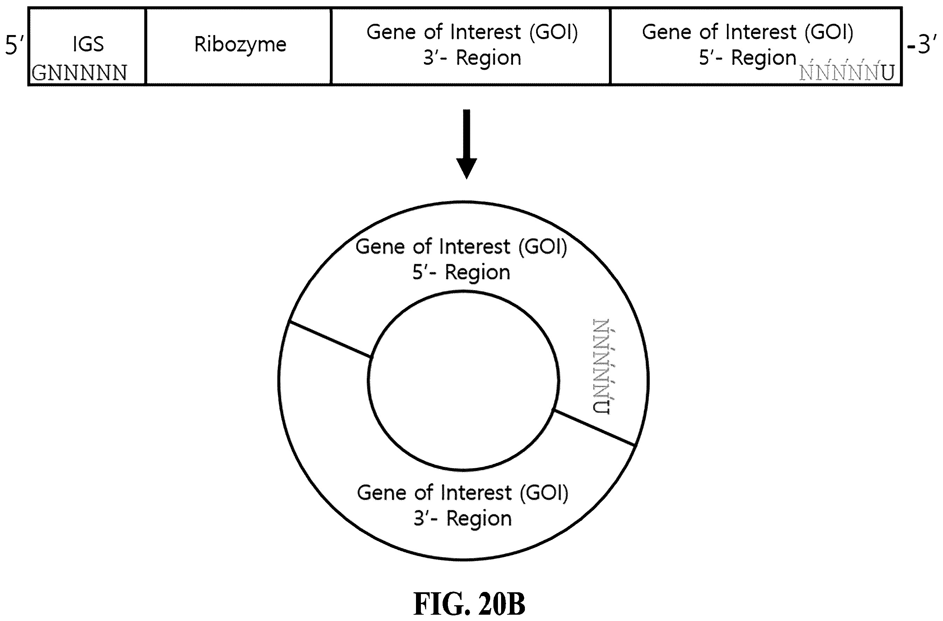

FIG. 20 is a simplified diagram of only the essential configuration of the self-circularization RNA construct. FIG. 20A shows that circRNA may be made from an RNA construct including only IGS, a ribozyme and GOI region, and a uracil base at the 3' end. FIG. 20B is a schematic diagram showing that circRNA may be made from simply IGS, a ribozyme, and RNA constructs of the GOI region when the uracil base is included at the 3' end of a GOI, in which the generated circRNA consists only of the GOI. In other words, FIG. 20B is a schematic diagram showing that when uracil is included after 5 unique nucleotide sequences at any site in the GOI, with that part as the 3' end, the rest of the 3' end of the GOI is sent to right behind the ribozyme, so that circular RNA consists only of the GOI without additional uracil.

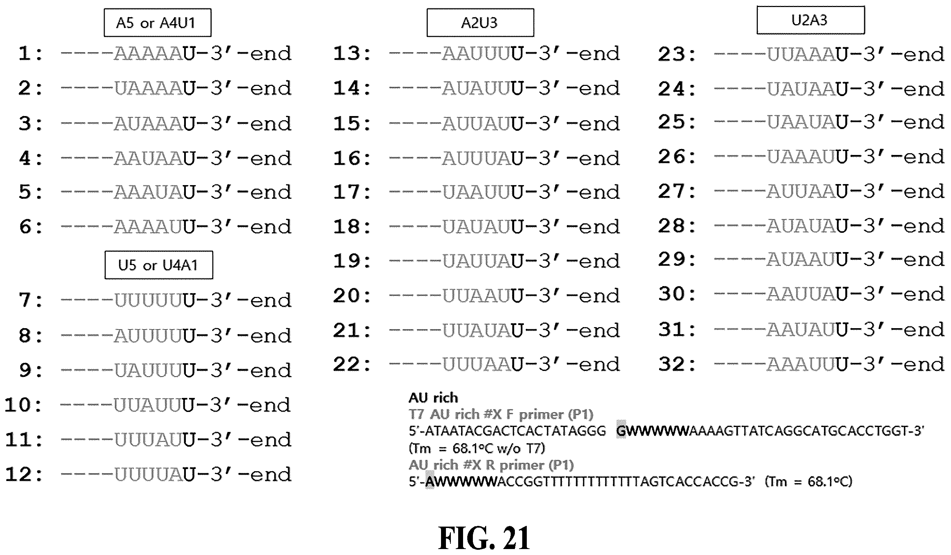

FIG. 21 shows the design of AU-rich target sites with various combinations.

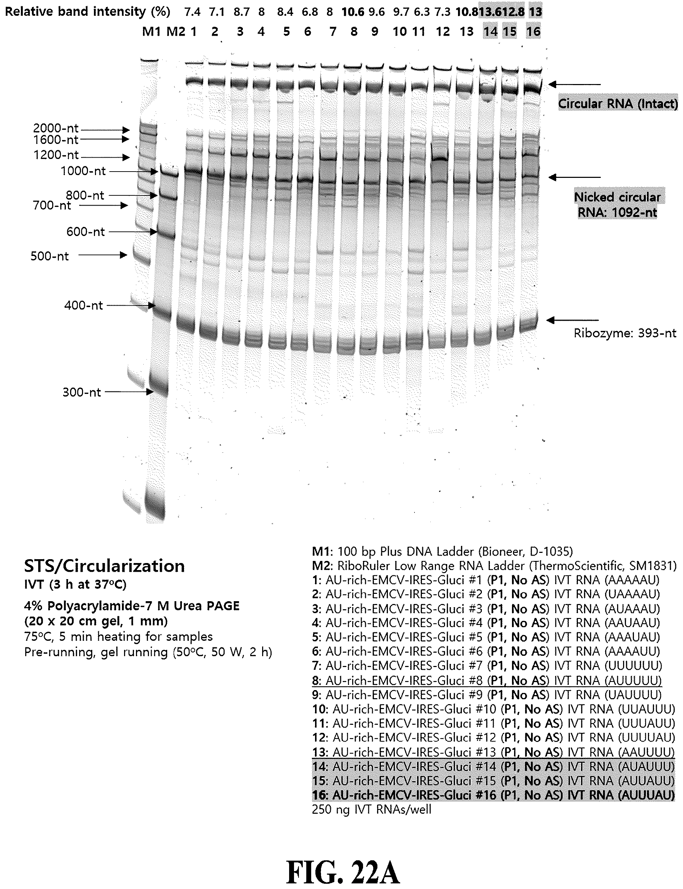

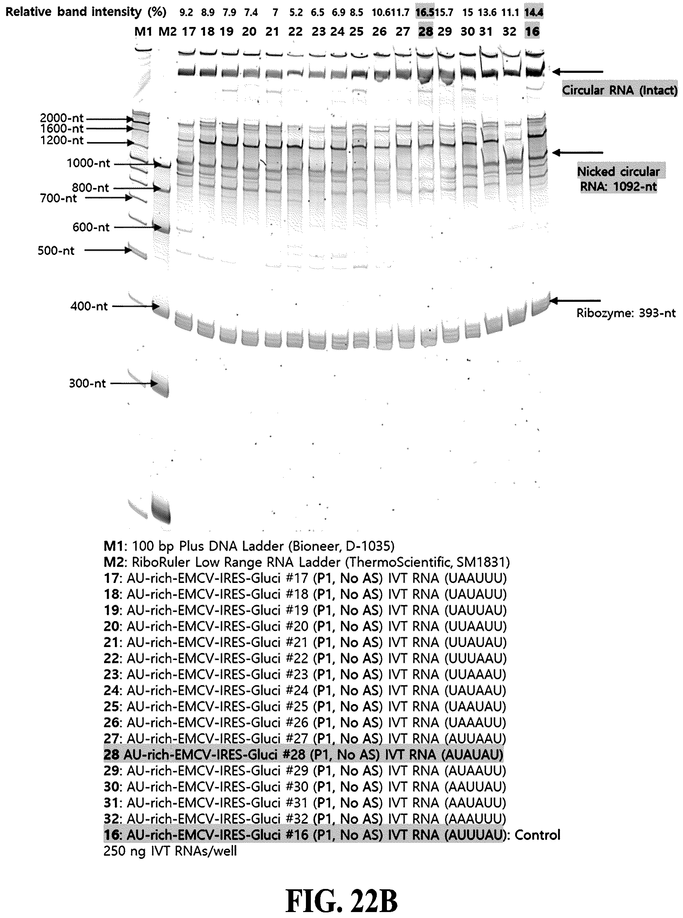

FIGS. 22A and 22B show electrophoresis results of samples obtained after in vitro transcription of a self-circularizing RNA expression vector having the AU-rich target sites shown in FIG. 21 and the corresponding IGS region, and show the results of denaturing PAGE confirming a circular RNA.

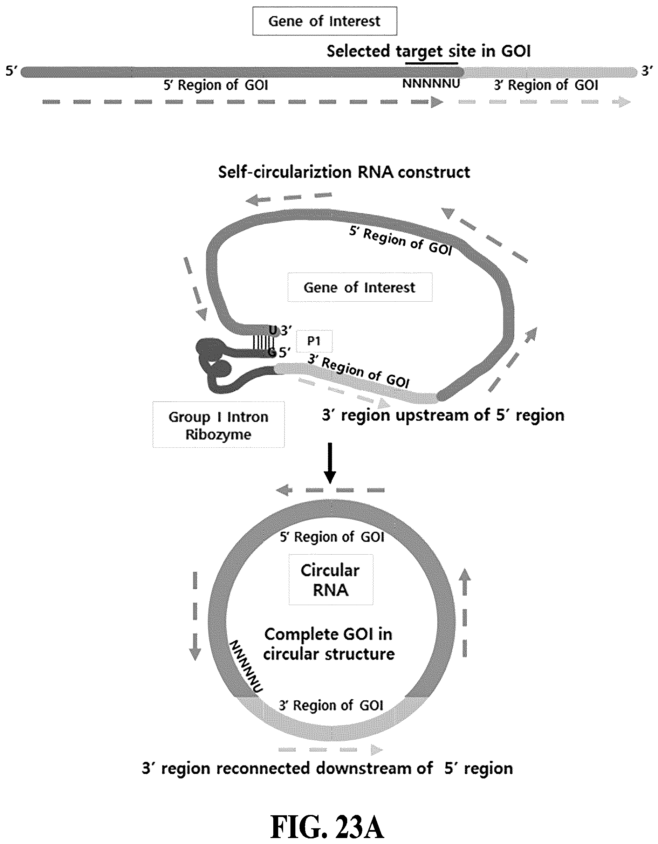

FIG. 23A is a simple schematic diagram illustrating that a circRNA consisting of only a GOI may be produced by selecting a target site within the gene of interest and reconstructing the GOI based on the target site.

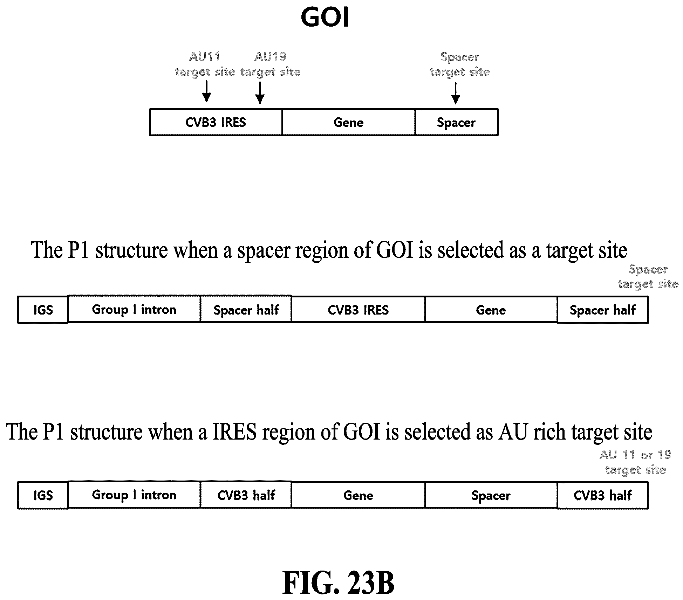

FIG. 23B is a schematic diagram of a reconstructed GOI structure in which the target site is selected within the gene of interest and the GOI is reconstructed based on the target site.

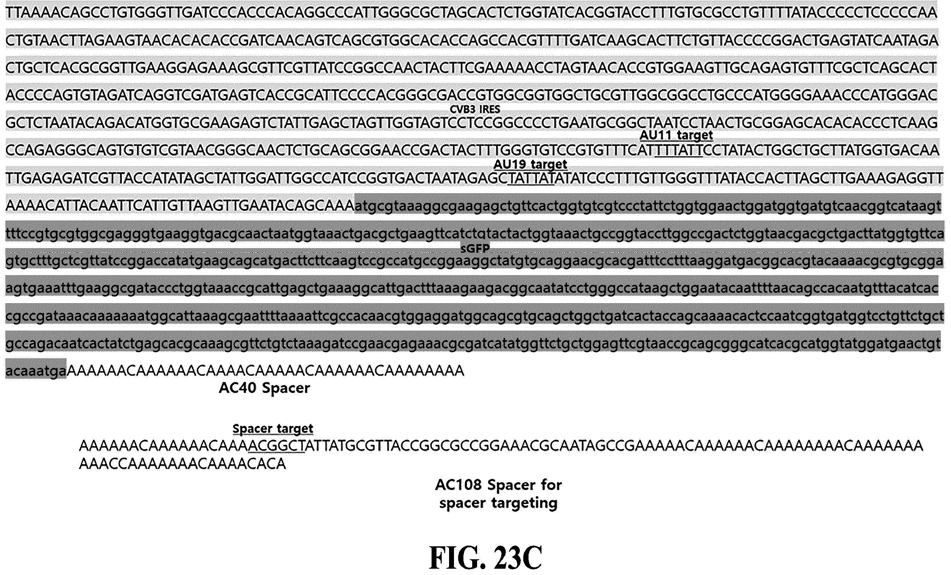

FIG. 23C shows the region of the target site in the nucleotide sequences of the gene of interest (CVB3 IRES-sGFP).

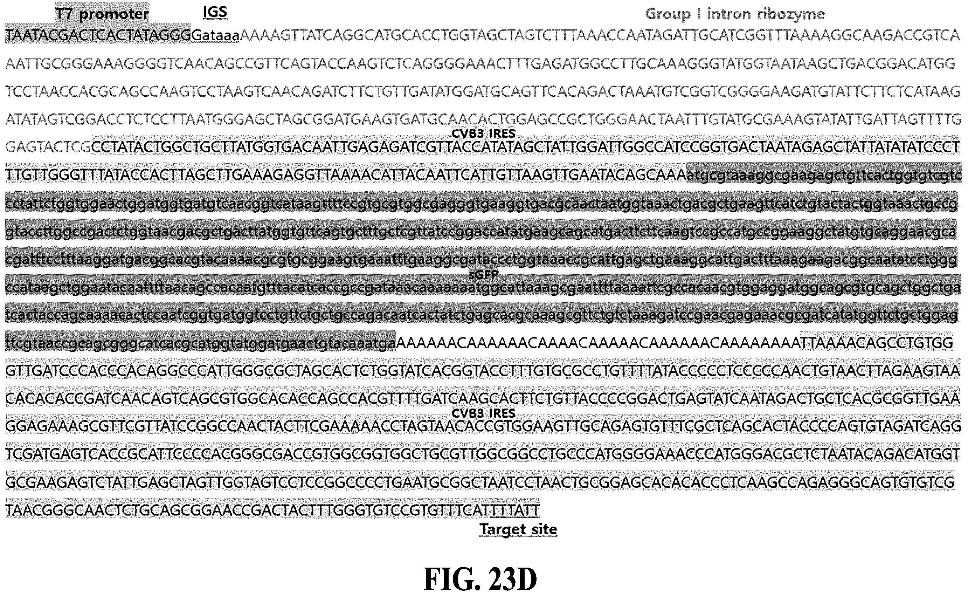

FIG. 23D shows each region in the DNA template for expression of a self-circularization RNA construct containing the reconstructed gene of interest (CVB3 IRES-sGFP).

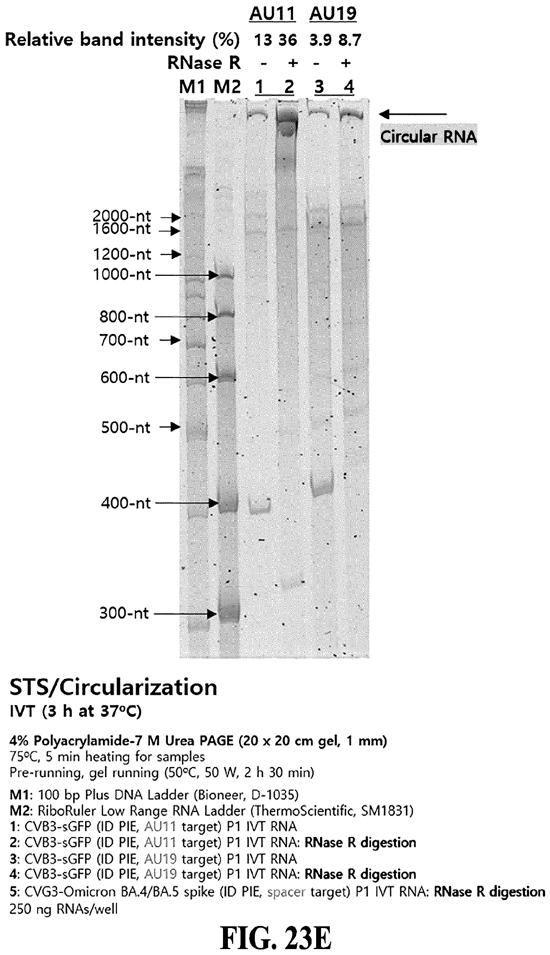

FIG. 23E shows the PAGE result after IVT was performed using a self-circularization RNA construct expression vector containing the reconstructed gene of interest (CVB3 IRES-sGFP).

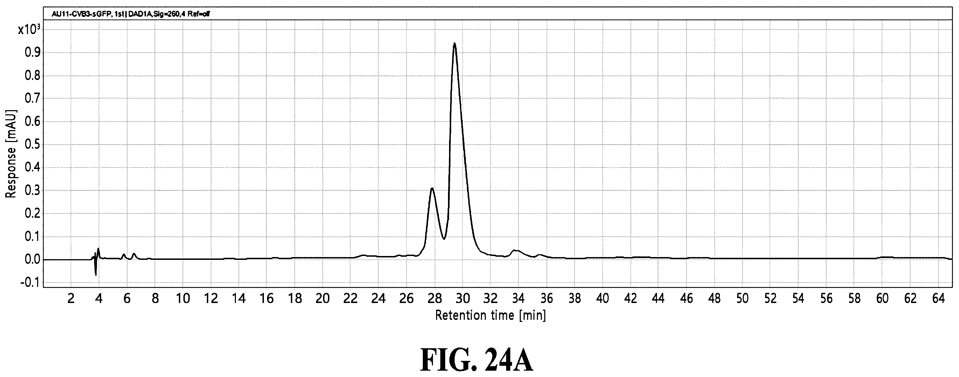

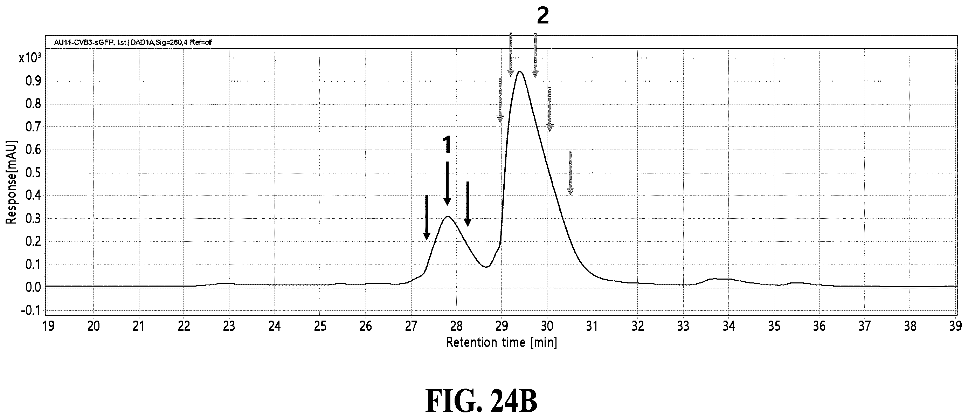

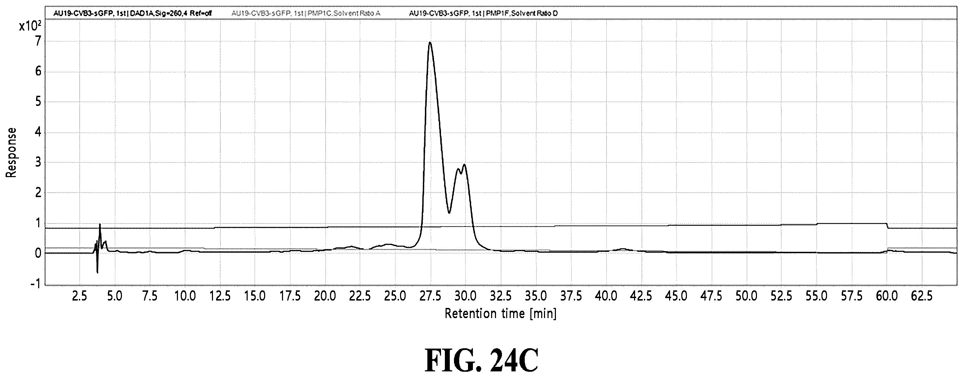

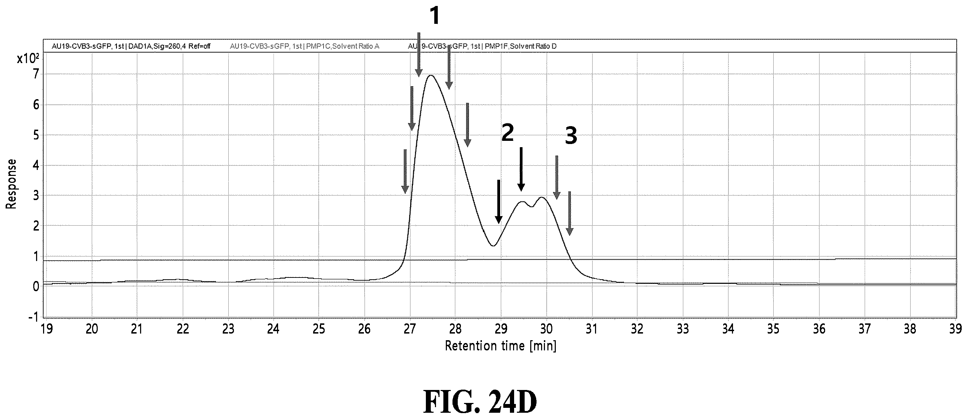

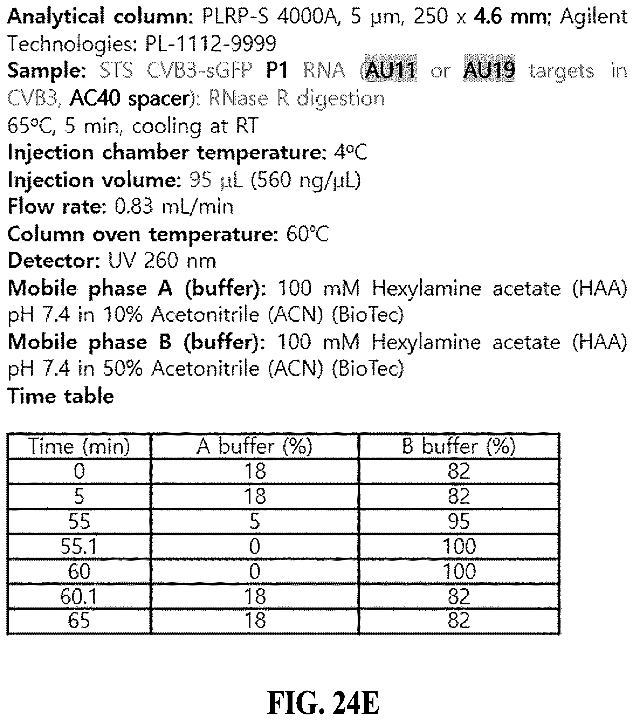

FIG. 24 shows the results of HPLC performed after IVT using a self-circularization RNA construct expression vector containing the reconstructed gene of interest (CVB3 IRES-sGFP).
Specifically, FIGS. 24A and 24B show the results of HPLC performed after IVT of a self-circularization RNA construct expression vector using a reconstructed gene of interest rearranged based on the AU11 target site in the gene of interest (CVB3 IRES-sGFP). FIGS. 24C and 24D show the results of HPLC performed after IVT of a self-circularization RNA construct expression vector using a reconstructed gene of interest rearranged based on the AU19 target site in the gene of interest (CVB3 IRES-sGFP). FIG. 24E shows the HPLC performance conditions.

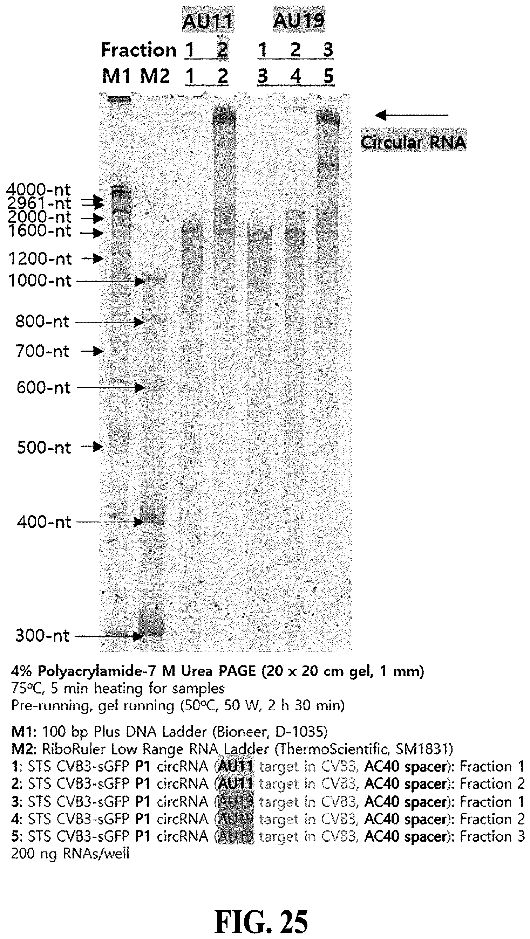

FIG. 25 shows the results of 4% denaturing PAGE performed on HPLC peaks from the IVT products using a self-circularization RNA construct expression vector containing the reconstructed gene of interest (CVB3 IRES-sGFP).

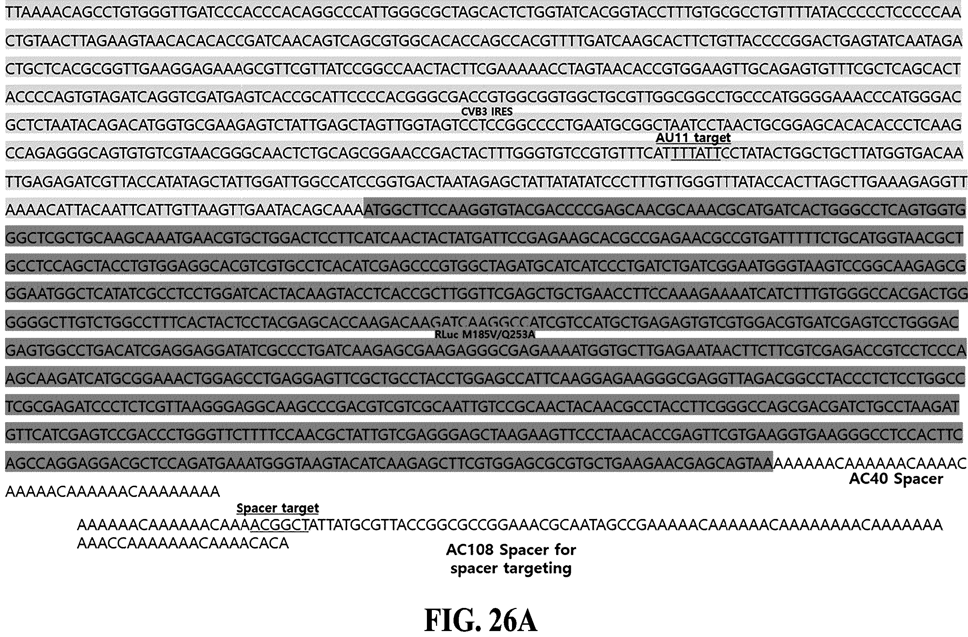

FIG. 26A shows the region of the target site in the nucleotide sequences of the gene of interest (CVB3 IRES-R.Luciferase mutant).

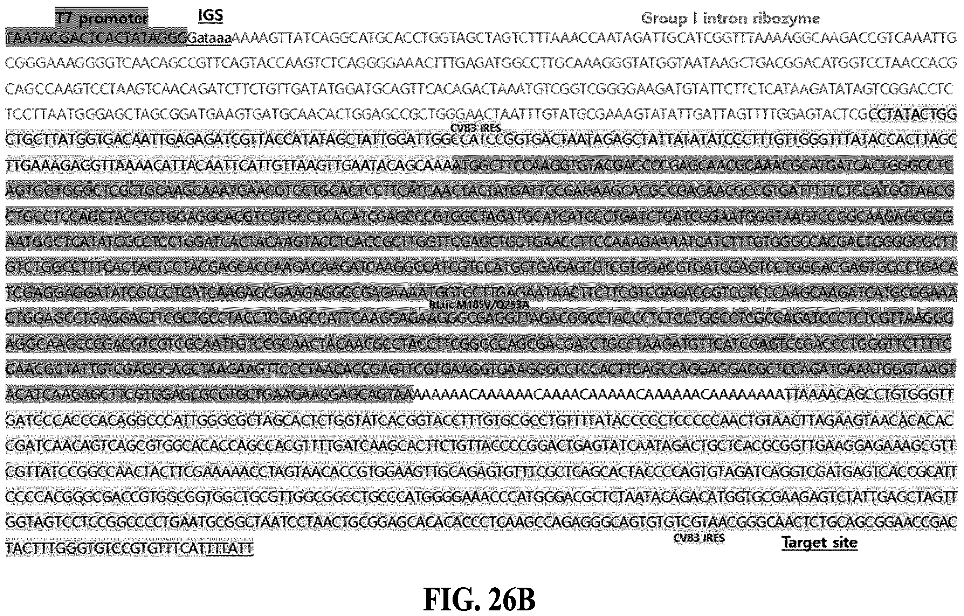

FIG. 26B shows each region in the DNA template for expression of a self-circularization RNA construct containing the reconstructed gene of interest (CVB3 IRES-R.Luciferase mutant).

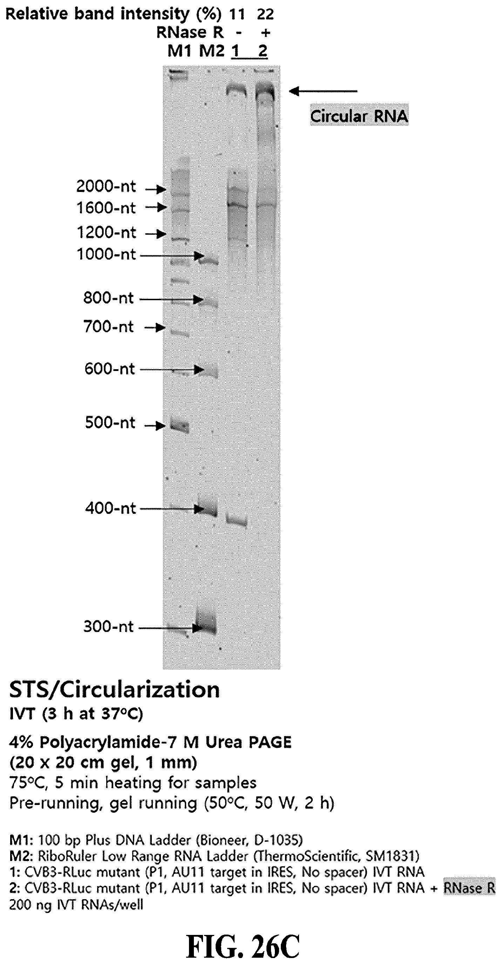

FIG. 26C shows the PAGE result after IVT was performed using a self-circularization RNA construct expression vector containing the reconstructed gene of interest (CVB3 IRES-R.Luciferase mutant).

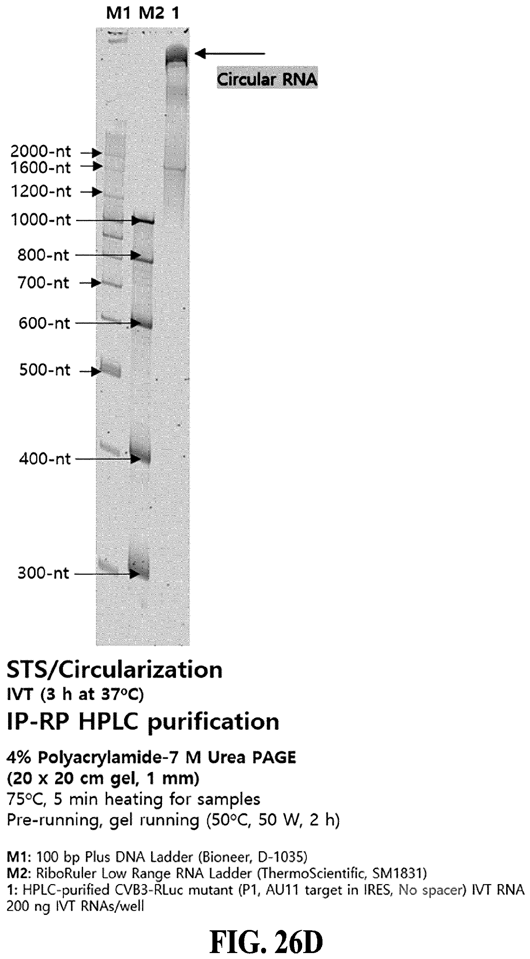

FIG. 26D shows the results of 4% denaturing PAGE performed on HPLC peaks from the IVT products using a self-circularization RNA construct expression vector containing the reconstructed gene of interest (CVB3 IRES-R.Luciferase mutant).

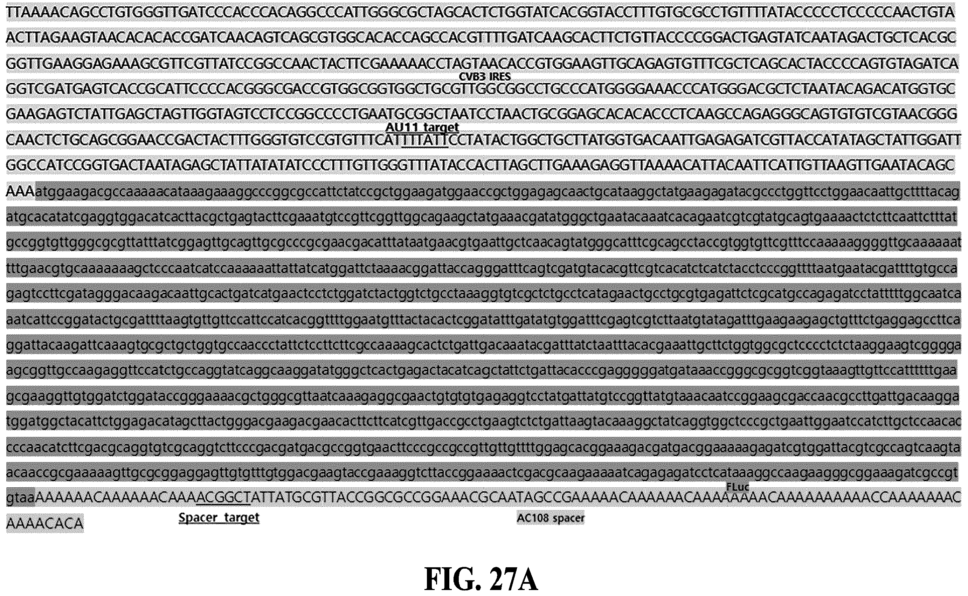

FIG. 27A shows the region of the target site in the nucleotide sequences of the gene of interest (CVB3 IRES-F.Luciferase).

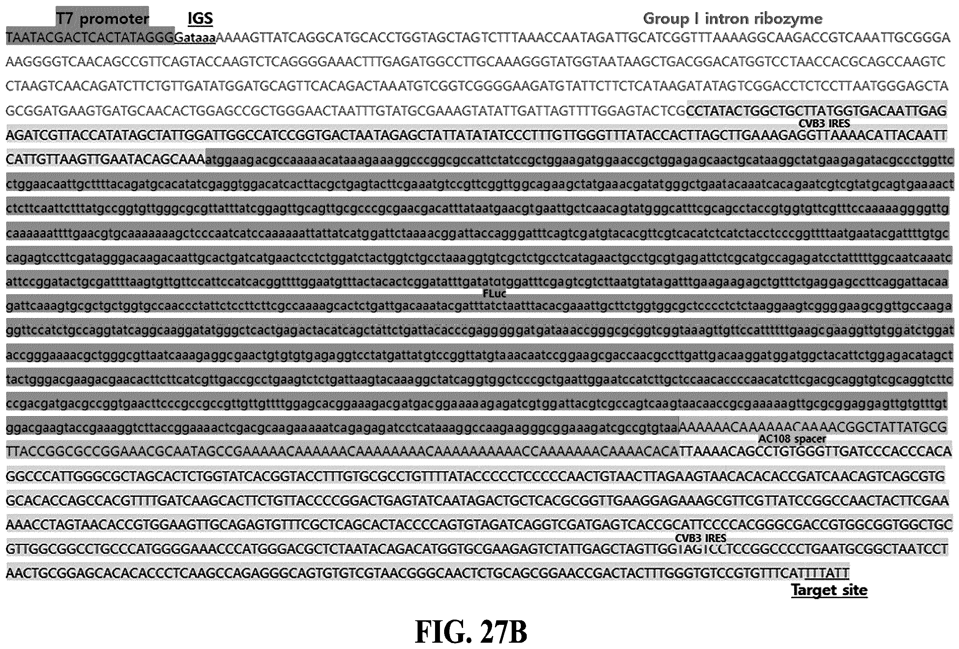

FIG. 27B shows each region in the DNA template for expression of a self-circularization RNA construct containing the reconstructed gene of interest (CVB3 IRES-F.Luciferase).

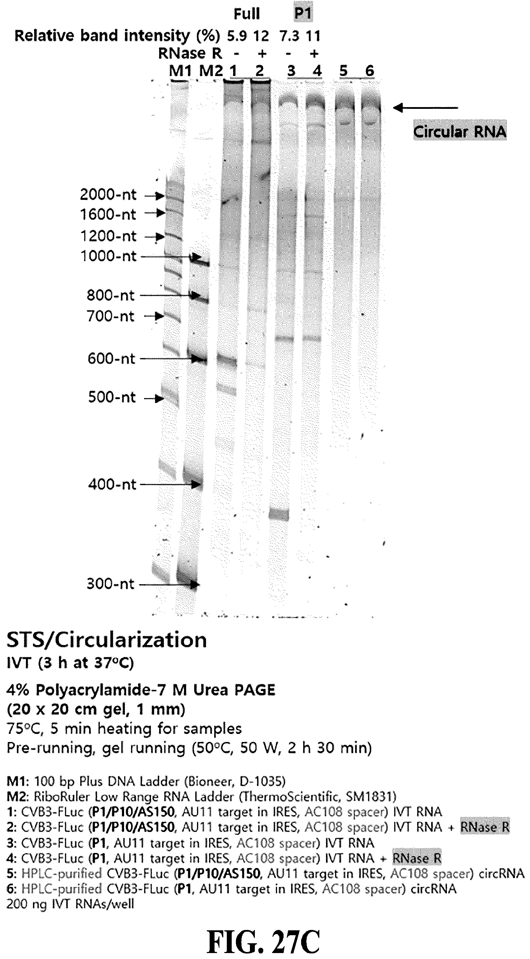

FIG. 27C shows the PAGE result after IVT was performed using a self-circularization RNA construct expression vector containing the reconstructed gene of interest (CVB3 IRES-F.Luciferase).

### BEST MODE FOR CARRYING OUT THE INVENTION

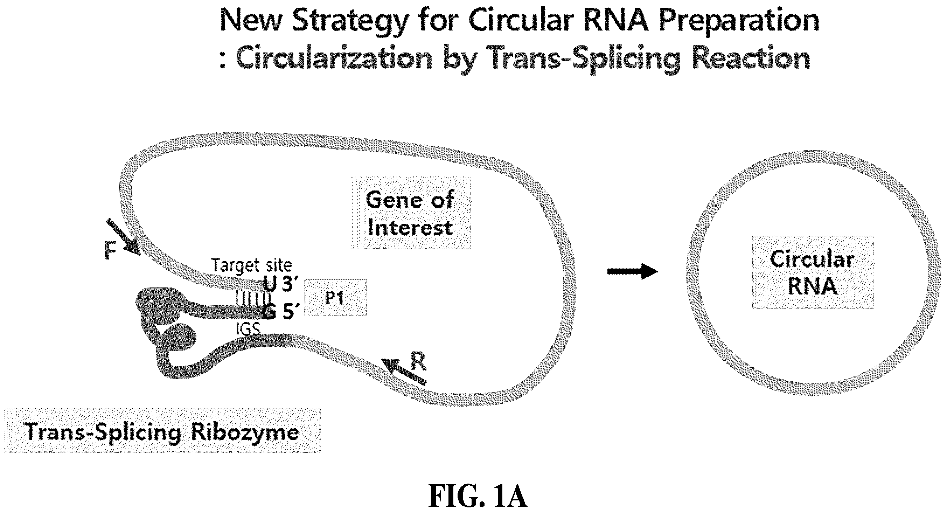

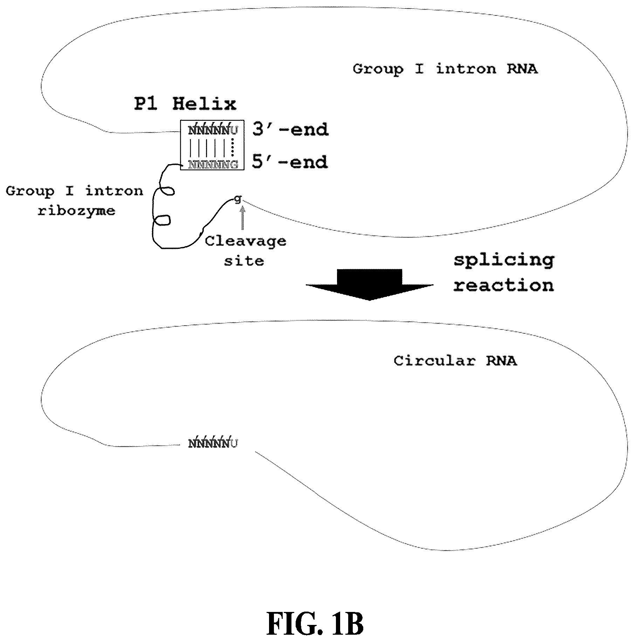

The present inventors used a trans-splicing ribozyme (T/S ribozyme) to prepare a circRNA, and devised a system in which the RNA containing a gene of interest performs a self-targeting and splicing reaction to be circularized (hereinafter referred to as "circularization system by self-targeting & splicing reaction") (FIGS. 1A and 1B).

A Group I intron ribozyme may induce trans-splicing by cleaving the target RNA through two successive trans-esterification reactions and then linking separate transcripts to each other at the cleaved 3' end.

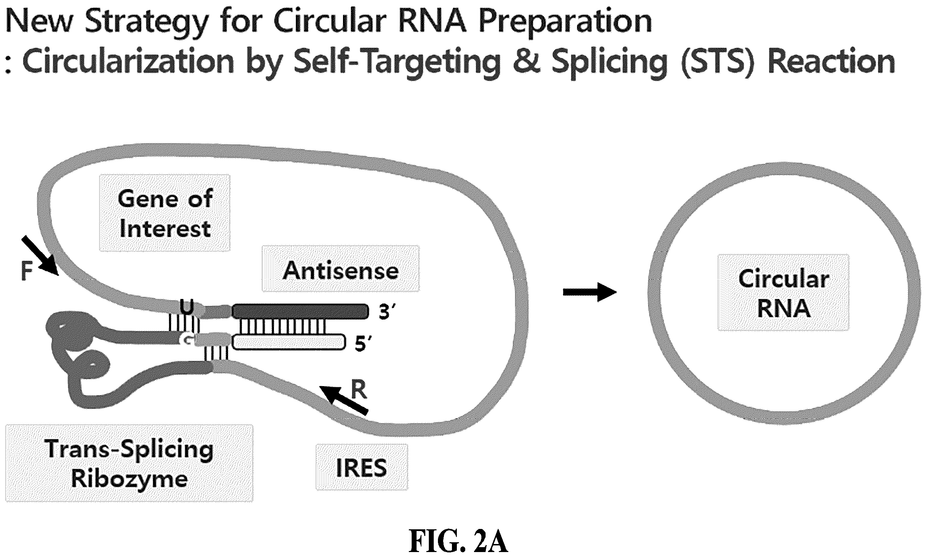

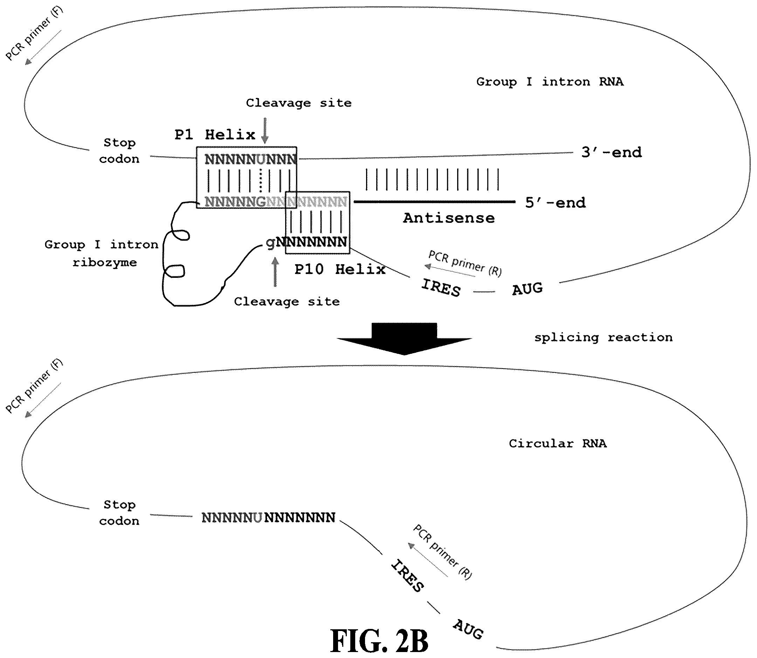

Accordingly, in the system of an embodiment of the present disclosure, an internal guide sequence (IGS) is configured in the 5' direction of a gene of interest (GOI) and a target site is configured in the 3' direction such that the IGS binds complementarily to the target site, and in the meantime, a guanine (G) : uracil (U) wobble base pair is formed, thereby inducing cleavage and splicing by the ribozyme located between the GOI and IGS so that a circRNA may be produced (FIGS. 2A and 2B).

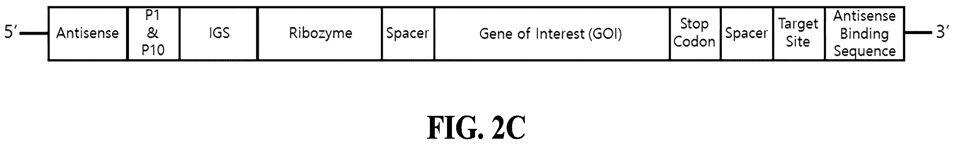

The present inventors focused on the previous study (Mol Ther. 2005 Nov; 12(5):824-34) for improving the trans-splicing efficiency of the Group I intron ribozyme, and as illustrated in FIG. 2C, designed a self-circularization RNA construct such that an AS (antisense sequence) region and an ABS (antisense binding sequence) capable of complementary binding to each other exist at the 5' and 3' ends of the self-circularization RNA construct, and P1 and P10 helixes are formed before/after the secondary structure of the ribozyme, and prepared a DNA template capable of expressing the RNA construct under a T7 promoter (Example 1).

Subsequently, a vector into which the DNA template was inserted was produced and transcribed in vitro (IVT), and then the formation of a circRNA was identified. As a result, it was found that the vector expresses a self-circularization RNA construct, and the RNA construct forms a monomeric circRNA through a self-targeting and splicing (STS) reaction immediately after transcription even without the addition of GTP (Example 2).

Furthermore, the present inventors tried to identify whether the self-circularization RNA construct expression vector may express the RNA construct of an embodiment of the present disclosure even in cells, and whether the expressed RNA may express a gene of interest loaded in the form of a circRNA. Specifically, a plasmid vector was produced by including IRES and a termination codon in a gene of interest to enable translation of the gene (transgene) in the cells and using gaussia luciferase as a transgene in the gene of interest, and the plasmid vector was transformed into cells to identify the production of a circRNA and luciferase activity. As a result, it was identified at the molecular level that the produced plasmid vector expressed the self-circularization RNA construct in the cells, the construct was circularized into a circRNA by a ribozyme, and the gene of interest was expressed in the circRNA (Example 3).

Subsequently, the present inventors attempted to optimize an RNA construct to efficiently form a circRNA through an immediate STS (direct STS) reaction by IVT.

First, the direct STS reaction rate according to a length of an AS region by IVT was identified through a specific experiment. Specifically, a self-circularization RNA expression vector was produced such that the lengths of the AS region and the ABS region were 50, 100, 150, 200, 250, or 300-nt, and the direct STS efficiency was identified after the vector was subjected to IVT. As a result, it was identified that the self-circularization efficiency was remarkably reduced at a length of 200-nt or more. Although the self-circularization efficiency was similar at 50, 100, and 150-nt lengths, it was identified that the in vitro transcription reaction itself decreased when the AS region and the ABS region of a 50-nt or 100-nt length were included. In terms of circRNA production efficiency, it was identified that the lengths of the AS region and the ABS region were preferably 150-nt (Example 5).

Like the circRNA production efficiency according to the lengths of the AS region and the ABS region was identified, the circRNA production efficiency according to the length and nucleotide sequence of a spacer region by IVT was identified. As a result, on the other hand, it was found that although its length and nucleotide sequence did not significantly affect the circRNA production efficiency, the linking of 30 adenines (A) was sufficient not to create a structural conflict that may occur due to a narrow gap between a ribozyme and the IRES in the case of the ribozyme and the EMCV IRES (Example 6).

In an embodiment of the present disclosure, a Group I intron ribozyme capable of a continuous trans-esterification reaction was used as a ribozyme. Group I intron ribozymes induce trans-splicing by linking separately existing transcripts to each other at the cleaved 3' end after cleavage of the target site, and link the 5' site of a GOI to the cleaved 3' end, which is not a separately existing transcript in a self-circularization RNA construct. The intention was to identify whether the P1 and P10 helix regions, known to increase trans-splicing efficiency, and the AS region and ABS region at both ends of the self-circularization RNA construct also had a positive effect on circularization efficiency.

Specifically, the present inventors produced a self-circularization RNA expression vector including only a P1 helix region, only P1 and P10 helix regions, or both the P1 and P10 helix regions and the AS region, and identified direct STS efficiency after IVT of the vector. As a result, surprisingly, it was found that the P10 helix region in the self-circularization RNA construct reduced the circRNA production efficiency when the P10 helix region was in the presence of the P1 helix region. Meanwhile, excellent circRNA production efficiency was exhibited even when only the P1 helix region was included without the AS region (Example 7).

Furthermore, the present inventors designed a self-circularization RNA construct such that only IGS forms the P1 helix, leaving only a gene of interest in the final product, circRNA, and identified whether an STS reaction occurs in various IGS sequences. As a result, surprisingly, even when only the IGS region formed the P1 helix, it was identified that the STS reaction of the self-circularization RNA construct expressed in DNA was induced, and furthermore, that the circRNA was formed even though the IGS region and the target site region were not complementary to each other. However, when the IGS region and the target site region are not complementary to each other, a non-specific reaction may occur at an undesired site, and thereby an unwanted product may be generated. The IGS region of 5'-GNNNNN-3' and the target site region of 5'-N'N'N'N'N'U-3' showed that the circRNA production efficiency increased as the ratio of nucleotide sequences capable of complementary binding to each other increased (Example 8-1). Furthermore, the present inventors tried to determine whether the efficiency of circRNA production could be increased in a specific sequence of IGS, based on the results of higher circularization efficiency in AU-rich regions. Therefore, the present inventors designed various combinations of AU-rich IGS except for bases forming wobble base pairs, constructed a DNA template expressing a self-circularization RNA structure to contain complementary target sites, and checked the degree of circRNA formation. As a result, it was possible for the present inventors to confirm circRNA was produced in all combinations of AU-rich IGS, and in particular, to confirm that the AU-rich IGS sequence consisting of two A bases and three U bases had a relatively high self-circularization efficiency (Example 8-2). From the above, the present inventors identified that it was possible to obtain a circRNA through an STS reaction only with the RNA construct illustrated in FIG. 1A, and the self-circularization RNA construct illustrated in FIG. 20A may form a circRNA consisting only of a gene of interest containing a U base at the GOI 3' end in the circRNA precursor as shown in the schematic diagram of FIG. 20B.

On the other hand, when the 3' end of the GOI ends with a U base, by designing the nucleotide sequence of the IGS region to be reverse-complementary to the GOI, the circRNA finally generated may be prepared such that it consists of only the GOI region (FIG. 20B). However, if the 3' end of the GOI does not end with a U base, it is difficult to produce a circRNA consisting of only the GOI. The present inventors confirmed that by selecting a target site within the GOI and reconstructing the GOI based on the target site, a circRNA consisting of only a GOI may be obtained even when the 3' end of the GOI does not end with a U base (Example 9).

When the target site is selected to be inside the gene of interest, the portion of the GOI located in the 3' direction based on the uracil base of the target site is referred to by the "3' Region of GOI", and the GOI region other than the GOI 3' region is referred to by the "5' Region of GOI". In this connection, the reconstructed gene of interest is designed by linking the 3' Region of GOI to the 5' Region of GOI in the 5' direction. That is, the reconstructed gene of interest has the structure of 5'-[3' Region of GOI]-[ 5' Region of GOI]-3', wherein [3' Region of GOI] and [5' Region of GOI] are directly linked.

To construct a circRNA for protein expression, the present inventors designed a gene of interest to include an IRES and a transgene encoding a protein, selected a target site within the gene of interest, then designed a reconstructed gene of interest so that the circRNA could consist of only the IRES and the transgene to construct a vector expressing a self-circularization RNA construct. As a result, it was confirmed that a circular RNA was produced. Specifically, a target site was selected within the gene of interest containing CVB3 as the IRES and containing a sGFP (superfolder GFP), RLuc M185V/Q253A, or FLuc gene as a transgene, and a reconstructed GOI was designed based on this. Then, a circular RNA precursor expression vector containing the reconstructed GOI was constructed, and IVT, PAGE, and HPLC were performed to confirm the circular RNA production and its yield (Examples 9-1 to 9-3). As a result, it was found that circular RNA precursors containing the reconstructed gene of interest produced a circular RNA regardless of the type of transgene, and that the selection of the target site affected the yield of circular RNA production.

The P1 helix is a helix structure formed through a complementary bond between the nucleotide sequence linked to the front end (5' direction) of the ribozyme and the nucleotide sequence in the 3' direction of the transcript in which conjugation is induced by the ribozyme, in the formation of the secondary structure of the Group I intron ribozyme, and the P10 helix is a helix structure formed through a complementary bond between a front end region of the ribozyme and a nucleotide sequence in the 5' direction of the transcript cleaved by the ribozyme.

In an embodiment of the present disclosure, the P1 helix may be formed through a complementary bond between the IGS at the 5' end and the target site at the 3' end in the self-circularization RNA construct of an embodiment of the present disclosure, and the P10 helix may be formed through a complementary bond between a nucleotide sequence extended at the 5' end and a nucleotide sequence linked to the rear end (3' direction) of the ribozyme.

Herein, the nucleotide sequence forming the P1 helix is referred to by the term "P1 region" or "P1 helix region", and similarly, the nucleotide sequence forming the P10 helix is referred to by the term "P10 region" or "P10 helix region".

In an embodiment of the present disclosure, the nucleotide sequence of the IGS region is 5'-GNNNNN-3', the nucleotide sequence of the target site region is 5'-N'N'N'N'N'U-3', and the P1 helix may be formed by complementarily binding the IGS region and the target site region to each other. In this connection, in order to exclude redundant expressions, the descriptions of the P1 helix region may be used interchangeably to describe the nucleotide sequence of the IGS region.

In an example embodiment of the present disclosure, the P1 helix may be formed including the nucleotide sequence naturally extended in the 5' direction of the IGS region. More specifically, the P1 helix may be constructed of the nucleotide sequence naturally extended in the 5' direction of the IGS region and the nucleotide sequence reverse complementary thereto, which are extended in the 3' direction of the target site. In this connection, herein, when the extended nucleotide sequence exists, the extended nucleotide sequence region is denoted by P1 to distinguish the extended nucleotide sequence from the IGS region. The same goes for the P10 helix.

In an embodiment of the present disclosure, the target site region is a nucleotide sequence that binds complementarily to the IGS region, which may overlap with the gene of interest (GOI) region depending on the design of the nucleotide sequence of the IGS region. In this connection, in order to specify the region that binds complementarily to the IGS region, the region that binds complementarily to the IGS region in the gene of interest is separately named "target site". In an embodiment of the present disclosure, the target site may overlap part or all of the gene of interest region, or may exist separately from the gene of interest.

In an embodiment of the present disclosure, the AS (antisense sequence) region is located at the 5' end of the self-circularization RNA construct and aims at hydrogen bonding with the nucleotide sequence of the ABS (antisense binding sequence) region located at the 3' end of the RNA construct. Hence, in an embodiment of the present disclosure, the AS region essentially coexists with the ABS region, and accordingly the absence of the AS region may also be understood as including the absence of the ABS region, and likewise, the RNA construct including the AS region may also be understood as including the RNA construct including the ABS region.

Meanwhile, regarding the circularization efficiency achieved by the STS reaction of the self-circularization RNA construct, as a result of identifying how the presence or absence of three types of configurations of the P1 region, P10 region, and AS region affects the efficiency, the present inventors identified that an amount of circRNA produced was higher in the order of when both the P1 and P10 regions and the AS region were included, when only the P1 region was included, and when only the P1 and P10 regions were included. Additionally, the present inventors identified that when only the P1 region was included the self-circularization RNA construct was also circularized with sufficient efficiency to generate a circRNA.

Accordingly, an embodiment of the present disclosure provides a self-circularization RNA construct, wherein only the P1 region is included. Herein, the self-circularization RNA construct including only the P1 region means that the P10 region and the AS region do not exist, and does not mean that configurations including regions other than the P10 region and the AS region are excluded. Herein, similarly, the self-circularization RNA construct including only the P1 region and the AS region means that the P10 region does not exist and does not mean that configurations including regions other than the P10 region are excluded.

Hereinafter, embodiments will be described in detail with reference to the accompanying drawings. However, various alterations and modifications may be made to the embodiments. Thus, the scope of the present application shall not be construed as limited or circumscribed by such embodiments. With respect to the embodiments, the scope of the present disclosure includes all changes, equivalents, and replacements.

The terminology used herein is for the purpose of describing particular embodiments only and is not intended to be limiting. The singular forms "a," "an," and "the" used herein include the plural forms as well, unless the context clearly indicates otherwise. The terms "comprises/comprising" and/or "includes/including" used herein, indicate the presence of stated features, integers, steps, operations, elements, and/or components, but do not preclude the presence or addition of one or more other features, integers, steps, operations, elements, components and/or groups thereof.

All terms including technical and scientific terms used herein have the meaning commonly understood by one of ordinary skill in the art to which the examples belong, unless otherwise defined herein. Terms defined in commonly-used dictionaries used herein, should be interpreted as having a meaning that is consistent with the meaning in the context of the relevant art and should not be interpreted in an idealized or overly formal sense unless expressly so defined herein.

In the descriptions of the embodiments making reference to the accompanying drawings, like reference numerals refer to like constituent elements and any repeated description related thereto has been omitted. In the descriptions of the embodiments, any detailed description of well-known related art that was deemed to make the present disclosure ambiguous such that it can be interpreted in more than one way, has been omitted.

### [Examples]

### Example 1. Design of Self-circularization RNA and Production of RNA Expression Vector

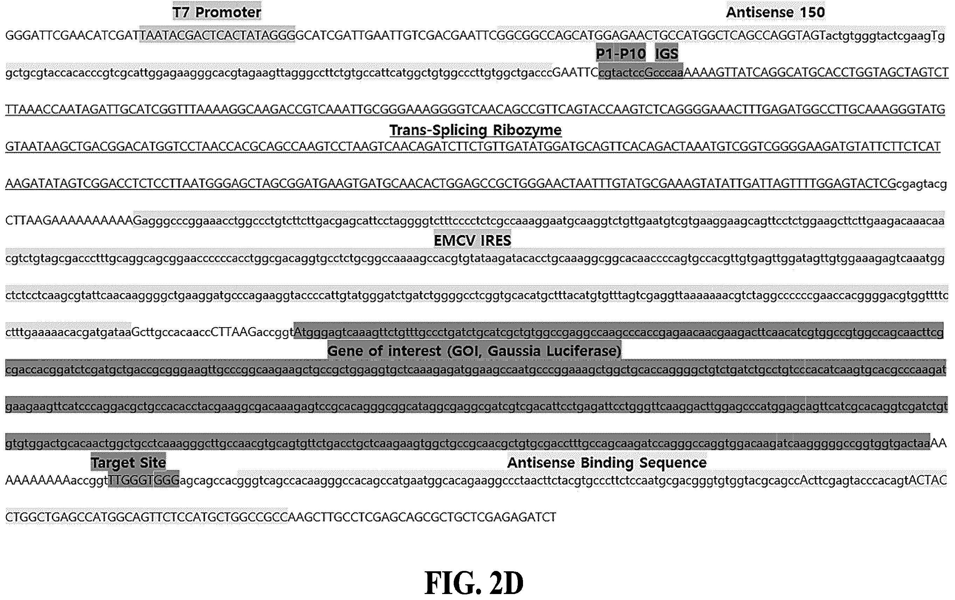

The self-circularization RNA was designed as shown in FIG. 2C, and a T7 promoter sequence was additionally included in the DNA template for its expression to enable in vitro transcription reaction (FIG. 2D). The DNA template was amplified by PCR using a T7 Circular Forward primer: 5'-GGGATTCGAACATCGATTAATACGACTCACTATAGGGGCATCGATTGAATTGT CGA-3' (Tm = 77.5°C) and a T7 Circular Reverse primer: 5'-AGATCTCTCGAGCAGCGCTGCTCGAGGCAAGCTT-3' (Tm = 79.4°C). The DNA template amplification product was inserted into the pTOP TA V2 cloning vector (Enzynomics) using a PstI restriction enzyme to produce a self-circularization RNA expression vector.

### Example 2. Identification of In Vitro Transcription and Self-Circularization

### 2-1. In Vitro Transcription (IVT)

The self-circularization RNA expression vector produced in Example 1 above was transcribed in vitro using a HiScribe T7 high yield RNA synthesis kit (NEB) according to the manufacturer's protocol. Specifically, a 20 uL scale (1 ug T7 DNA template, 1 X Reaction buffer, 10 mM each ATP, UTP, CTP, GTP, T7 RNA polymerase mix 2 ul) was reacted for 3 hours at 37°C, then 29 uL of nuclease-free water was added. Afterwards, 1 uL of RNase-free DNase I (10 U/ul) was added thereto and then the mixture was reacted at 37°C for 30 minutes to induce transcription, after which an immediate circularization (direct STS) reaction was induced.

Subsequently, an additional circularization reaction was induced to identify the stage in which the self-circularization reaction was completed. Specifically, in order to induce the first self-targeting and splicing (1^{st} STS) reaction, 28 uL of nuclease-free water, 20 uL of 5X STS buffer (50 mM Hepes (pH 7.0), 150 mM NaCl, 5 mM MgCl₂), and 2 uL of 100 mM GTP (final 2 mM) were further added to make a volume of 100 uL, after which a self-circularization reaction was performed at 37°C for 1 hour. Then, heating was performed at 55°C for 15 minutes, and after the heating, column purification was performed using a Monarch RNA cleanup kit (NEB). A second STS (2nd STS) reaction was induced by adding 20 uL of 5X STS buffer, 100 mM GTP (final 2 mM), and 28 uL of nuclease-free water to 50 uL of the column-purified sample again to make a final volume of 100 uL and a reaction was performed at 37°C for 3 hours. After the reacting, heating was performed at 55°C for 8 minutes, then column purification was performed using a Monarch RNA cleanup kit (NEB), and the concentration was measured using Nanodrop (Thermo Fisher Scientific product) equipment.

Meanwhile, in order to remove linear RNA from the reaction product, after the first and second STS reactions were performed, some of the column-purified samples were treated with RNase R. Specifically, 10 uL of 10X RNase R reaction buffer (10X: 0.2 M Tris-HCl pH 8.0, 1 M KCl, 1 mM MgCl₂) and 20 units of RNase R were added to column-purified IVT RNA of up to 100 ug (adjusting the volume to 100 uL with water), and the mixture was reacted at 37°C for 30 minutes, and then 10 units of RNase R were further added, and thereby the reaction was performed for another 30 minutes. Then, column purification was performed using a Monarch RNA cleanup kit (NEB) and a concentration was measured with Nanodrop equipment.

250 ng of each of the samples treated with RNase R on RNA obtained from each STS stage and RNA obtained from each stage was mixed in a ratio of 1:1 with 10 M Urea-BPB (IX TBE) dye, heated at 75°C for 5 minutes, and then a 4% polyacrylamide-7 M urea denaturing PAGE (2 hours of electrophoresis at 50 W conditions while maintaining a temperature of 50°C) was performed. Then, the gel was stained with an SYBR Gold Nucleic Acid Stain product (Thermo Fisher Scientific) and analyzed using an ImageQuant 800 (Cytiva product).

As a result, as can be seen in FIG. 3, by the RNase R treatment, RNA bands that were not well cleaved by RNase R and became enriched were revealed (Candidate 1). In addition, bands that were clearly observable despite the RNase R treatment were identified (bands presumed to be Candidate 2 and nicked circular RNA). In addition, it had already been identified that even without additional 1^{st} and 2^{nd} STS reactions, substances presumed to be circRNAs were sufficiently prepared only by a direct STS reaction, that is, an in vitro transcription reaction.

### 2-2. Identification of Self-Circularization

Subsequently, RT-PCR sequencing was performed to verify that a Candidate 1 was a circRNA among the RNA bands not cleaved by RNase R from the PAGE results. Specifically, RT-PCR was performed using a circular RNA sample purified by ethanol precipitation by cutting and crushing the band at the Candidate 1 position in the PAGE results and eluting the band in water at 37°C for 3 hours up to 16 hours and using a linear RNA that does not allow circularization due to the absence of a ribozyme site and an antisense site, as controls, and using primers capable of PCR amplification only when circRNAs were made.

For reverse transcription (RT), OneScript Plus RTase (Abm) was used, and 125 ng of each RNA (a sample of RNAs having or not having been treated with RNase R presumed to be control RNAs and circular RNAs) was heated at 70°C for 5 minutes. Thereafter, the sample was put on ice, and to the sample were added 5X RT buffer 4 uL, 10 mM dNTP mix 1 uL, 2 uM reverse primer (Circular STS R) 1 uL, and OneScript Plus RTase 200 U in an orderly manner. Then, the sample was reacted at 50°C for 15 minutes in a final volume of 20 uL, heated at 95°C for 5 minutes to inactivate the enzyme, and then stored on ice.

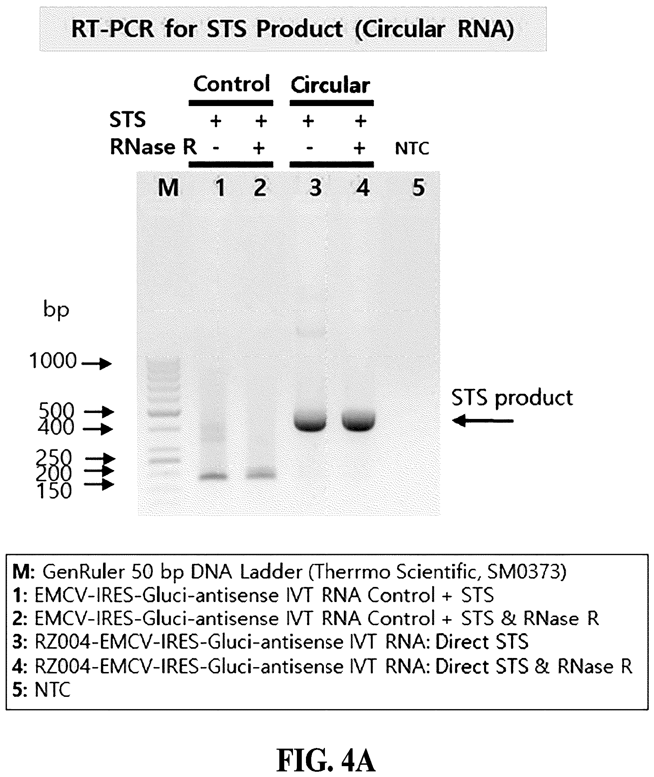

Subsequently, PCR was performed using AccuPower Taq PCR premix (Bioneer), 2 uL of an RT sample and 1 uL each of 20 uM of Circular STS F (5'-CCCTGAGTGGCTGAGCTCAGG-3') and Circular STS R (5'-CAGCAAGCATACTAAATTGCCAG-3') were added and the volume was adjusted to a volume of 20 uL with water. PCR amplification was performed under the conditions of 95°C for 1 minute, [95°C for 30 seconds, 65°C for 30 seconds, and 72°C for 30 seconds], 35 cycles, and 72°C for 5 minutes. 5 uL of a PCR product was put into 1 uL of 6X DNA loading dye, electrophoresed at 150 V for 35 minutes, and images were analyzed using a gel imaging system (Davinch-Gel product of Youngin Scientific). The expected length of the STS PCR product was 479 bp. When analyzed by a GeneRuler 50 bp DNA ladder (Thermo Fisher Scientific) on 1.5% agarose gel (Intron Bio's product, RedSafe Nucleic Acid Staining Solution, is contained in a concentration of 1X), only in the case of an RNA sample presumed to be circular RNA, a specific PCR product of the predicted size was observed regardless of RNase R treatment (FIG. 4A).

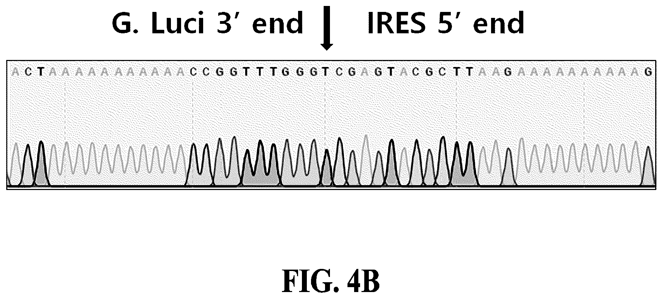

The PCR product was isolated and purified according to the manufacturer's protocol using a gel extraction kit (Cosmogenetech product) from the obtained band of the expected size, cloned using a TOPcloner TA-Blunt kit (Enzynomics product), and transformed into DH5alpha E. coli (Chemically competent E. coli, Enzynomics product) to obtain E. coli colonies from LB-Agar (including Kanamycin) plates. Plasmid DNA was extracted and purified according to the manufacturer's protocol using a DNA purification kit (Cosmogenetech product). Sequencing (Sanger sequencing service of Cosmogenetech, using M13R (-40) or M13F (-20) universal primer provided by the company) was requested. From the sequencing results, it was identified that 3' of Gaussia Luciferase and 5' of IRES were exactly linked at an STS junction site (FIG. 4B).

From the above mentioned results, it can be seen that a Candidate 1 is a circular RNA.

### 2-3. Revalidation of Self-Circularization

It was identified that a Candidate 1 is a circRNA through RT-PCR, but it was determined that theoretically, the possibility that the Candidate 1 can be in the form of a dimer may not be excluded. Accordingly, a nicking test was performed to revalidate that a Candidate 1 is a circRNA.

MgCl₂ was mixed with purified circular RNA Candidates 1 and 2 (100 ng) to achieve final concentrations of 0, 2.5, and 5 mM, respectively. The sample present in the final 10 uL of water was heated at 65°C for 30 minutes and then stored on ice for a while. Afterwards, the sample was mixed with 10 uL of 10 M Urea-BPB (IX TEB) loading dye.

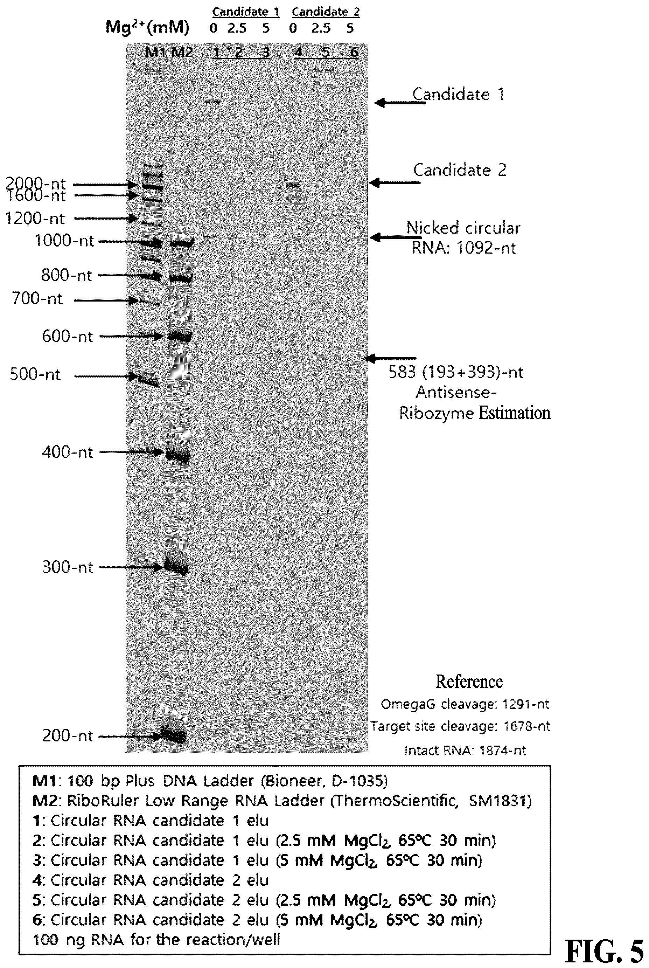

The mixture was heated at 75°C for 5 minutes. After the heating, 4% polyacrylamide-7 M urea denaturing PAGE (2 hours of electrophoresis at 50 W conditions while maintaining a temperature of 50°C) was performed. Gels were stained with an SYBR Gold Nucleic Acid Stain product (Thermo Fisher Scientific) and analyzed using an ImageQuant 800 (Cytiva product). The results are shown in FIG. 5.

For Candidate 1, it was identified that in the absence of Mg²⁺, a band with a size corresponding to a slightly nicked circular RNA was included (1092-nt), and in the case of a 2.5 mM Mg²⁺ condition, the band at the position of Candidate 1, which is believed to be the position of a circular RNA, was decreased. In addition, it was identified that a band of nicked circular RNA size was still present. In the case of a 5 mM Mg²⁺ condition, it was identified by hydrolysis that even the nicked circular RNA band disappeared. Thus, it was observed that Candidate 1, that is, circular RNA, started at the size of 1092-nt as expected for a monomer when nicking occurred (2.5 mM Mg²⁺) and eventually the Candidate 1 completely degraded (5 mM Mg²⁺). Accordingly, it was identified again that the Candidate 1 is a circular RNA and a monomer.

On the other hand, the size of Candidate 2 is similar to the size of intact RNA, 1874-nt (around 2000-nt of the marker). Unlike Candidate 1, under mild nicking conditions of 2.5 mM Mg²⁺, it was identified that the band of 1092-nt, the size of the nicked circular RNA, was not generated and disappeared, and thus that Candidate 2 was not a circular RNA.

### Example 3. Identification of Intracellular Transcription and Self-Circularization

In Example 2, it was identified that the self-circularization RNA expression vector produced in Example 1 was transcribed in vitro to form a circRNA. Accordingly, the intention was to identify whether the vector operated in the same way in cells, and furthermore, whether the gene of interest included in the circRNA was expressed smoothly in the cells.

For expressing a gene of interest in cells, the gene of interest was designed with the structure of 5'-EMCV IRES-transgene-stop codon-3', and so the expressed gene of interest could be easily identified, gaussia luciferase (G.luci) was used as a transgene.

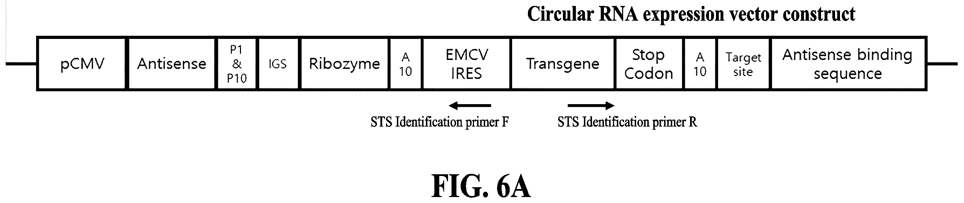

A self-circularization RNA construct including the aforementioned gene of interest was designed, and a DNA template, which may express the same, was inserted into a plasmid to be expressed under a pCMV promoter (FIG. 6A). The plasmid vector was transfected into 293A cells, the generation of a circRNA was identified by RT-PCR and sequencing, and the expression of transgene was identified by performing a luciferase activity assay.

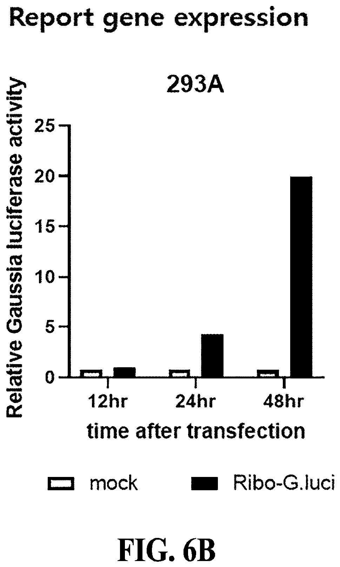

Specifically, 293A cells were seeded at 2×10⁵/well in 6-well plates and after 24 hours were transformed into the plasmid vector using a lipofectamine 2000 transfection reagent. After 6 hours of transformation, a culture medium was replaced. After the transformation, 100 UL of culture medium was collected at 12, 24, and 48 hours to measure G.luci activity. It was identified that G.luci activity was detected in the culture medium and the activity increased over time, showing that the transgene G.luci gene was expressed in the vector introduced into the cells (FIG. 6B).

It was investigated at the molecular level through RT-PCR and sequencing whether transgene expression was caused by the formation of a circRNA. 48 hours after transformation, total RNA was extracted from the 293A cells introduced with the plasmid vector using a trizol reagent, and then RT-PCT was performed, through which it was identified that circular RNA was generated.

For reverse transcription (RT), OneScript Plus RTase (Abm product) was used, and 1 ug of total RNA was heated at 70°C for 5 minutes and then placed on ice. Thereafter, 5X RT buffer 4 uL, 10 mM dNTP mix 1 uL, 2 uM reverse primer (Circular STS R) 1 uL, and OneScript Plus RTase 200 U were added thereto in an orderly manner. Then, the mixed sample was reacted at 50°C for 15 minutes in a final volume of 20 uL, heated at 95°C for 5 minutes to inactivate the enzyme, and then stored on ice.

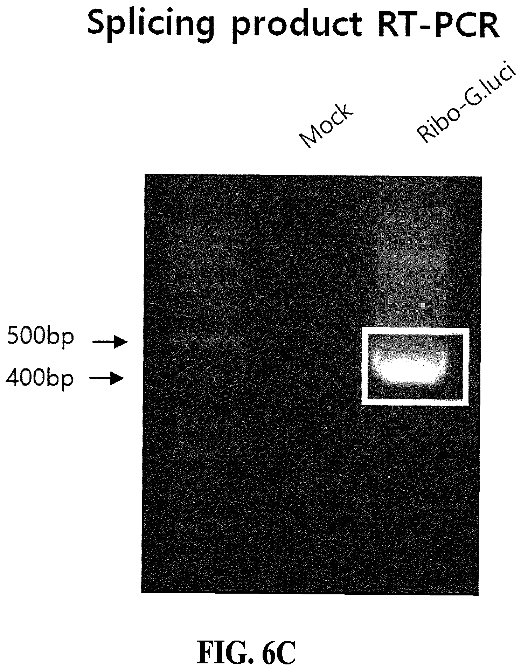

Subsequently, PCR was performed using AccuPower Taq PCR premix (Bioneer product), 2 uL of an RT sample and 1 uL each of 20 uM of Circular STS primer F (5' - caaggacttggagcccatggagcag - 3') and primer R (5' - tgtgccgcctttgcaggtgtatc - 3') were added, the volume was adjusted to a volume of 20 uL with water, and PCR amplification was performed under the conditions of 95°C for 1 minute, [95°C for 30 seconds, 65°C for 30 seconds, and 72°C for 30 seconds] 35 cycles, and 72°C for 5 minutes. The expected length of the STS PCR product was 479 bp. When analyzed by a GeneRuler 50 bp DNA ladder (Thermo Fisher Scientific product) on 1.5% agarose gel (Intron Bio product, RedSafe Nucleic Acid Staining Solution, is contained in a concentration of 1X), a specific PCR product of the predicted size was identified only when circular RNA was present. In this connection, 5 uL of a PCR product was put into 1 uL of 6X DNA loading dye, the product was electrophoresed at 150 V for 35 minutes, and images were analyzed using a gel imaging system (Davinch-Gel product of Youngin Scientific) (FIG. 6C).

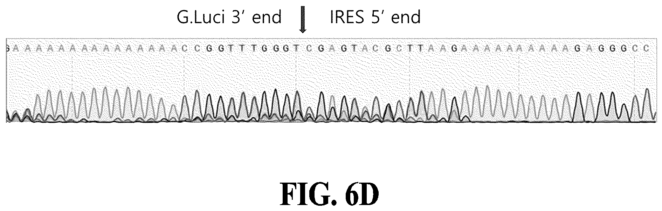

In addition, the PCR product was isolated and purified according to the manufacturer's protocol using a gel extraction kit (Cosmogenetech product) from the obtained band of the expected size, cloned using a TOPcloner TA-Blunt kit (Enzynomics product), and transformed into DH5alpha E. coli (Chemically competent E. coli, Enzynomics product) to obtain E. coli colonies from LB-Agar (including Kanamycin) plates. Plasmid DNA was extracted and purified according to the manufacturer's protocol using a DNA purification kit (Cosmogenetech product) and manual. Sequencing (Sanger sequencing service of Cosmogenetech, using M13R (-40) or M13F (-20) universal primer provided by the company), was requested. From the sequencing results it was identified that 3' of Gaussia Luciferase and 5' of IRES were exactly linked nucleotide sequences at an STS junction site (FIG. 6D).

From the above, it was identified at the molecular level that the produced plasmid vector expressed the self-circularization RNA construct in the cells, the construct was circularized into a circRNA by a ribozyme, and the gene of interest was expressed in the circRNA.

### Example 4. Purification of circRNA

### 4-1. Identification of circRNA Purification Potential by Performing HPLC

In order to minimize the occurrence of immunogenicity in the production of a circRNA for human injection, analysis and purification using HPLC were performed. Specifically, an Agilent 1290 Infinity II Bio UHPLC system was used. The analysis conditions are shown in FIG. 7A. For the analysis, the gradient conditions of [Analytical] were used, and for sample fractionation the conditions of [Fraction collection] were used.

When RNA purified only with a column (a Monarch RNA cleanup kit (NEB)) after IVT (FIG. 7B) and RNA treated with RNase R (FIG. 7C) were analyzed, an increasing peak in the RNase R-treated sample was found, indicating that a circRNA could be isolated by HPLC without RNase R treatment.

### 4-2. Purification of circRNA by Performing HPLC

Through HPLC, fractionation was performed using the gradient shown in the [Fraction collection] conditions, and each fraction was obtained as shown in FIG. 8A. In the same manner as before, 4% denaturing PAGE was performed by loading 200 ng each of fractions 7 and 10 to 13 with clear peaks.

As a result, as can be seen in FIG. 8B, clean circular RNA was isolated and purified from fraction 12.

### Example 5. Antisense Sequence (AS) Region Optimization

The intention was to identify the effect of the lengths of an antisense sequence (AS) region and a reverse-complementary antisense binding sequence (ABS) region on the immediate circularization reaction during an in vitro transcription process. Accordingly, a DNA template having different lengths of the AS region and ABS region of 50, 100, 150, 200, 250, or 300-nt was prepared (FIG. 9). A vector capable of expressing each RNA construct was produced in a manner like the one used for Example 1, and in vitro transcription was performed at 37°C for 3 hours in a manner like the one used for Example 2. The degree of immediate STS reaction was compared and identified through relative band intensity by performing PAGE on 4% polyacrylamide-7 M urea gel (20×20 cm, 1 mm).

The nucleotide sequences of each AS region are shown in Table 1 below.

**[Table 1]**

| Sections | Nucleotide sequences (5' --> 3') |
|---|---|
| AS50 | AAGTTAGGGCCTTCTGTGCCATTCATGGCTGTGGCCCTTGTGGCTGACCC |
| AS100 | ACTCGAAGTGGCTGCGTACCACACCCGTCGCATTGGAGAAGGGCACGT AGAAGTTAGGGCCTTCTGTGCCATTCATGGCTGTGGCCCTTGTGGCTGA CCC |
| AS150 | GGCGGCCAGCATGGAGAACTGCCATGGCTCAGCCAGGTAGTACTGTGG GTACTCGAAGTGGCTGCGTACCACACCCGTCGCATTGGAGAAGGGCAC GTAGAAGTTAGGGCCTTCTGTGCCATTCATGGCTGTGGCCCTTGTGGCT GACCC |
| AS200 | AGCGTGAGGAAGTTGATGGGGAAGCCCAGCACGATCAGCAGAAACATG TAGGCGGCCAGCATGGAGAACTGCCATGGCTCAGCCAGGTAGTACTGT GGGTACTCGAAGTGGCTGCGTACCACACCCGTCGCATTGGAGAAGGGC ACGTAGAAGTTAGGGCCTTCTGTGCCATTCATGGCTGTGGCCCTTGTGG CTGACCC |
| AS250 | GGATGTAGTTGAGAGGCGTGCGCAGCTTCTTGTGCTGGACGGTGACGTA GAGCGTGAGGAAGTTGATGGGGAAGCCCAGCACGATCAGCAGAAACAT GTAGGCGGCCAGCATGGAGAACTGCCATGGCTCAGCCAGGTAGTACTG TGGGTACTCGAAGTGGCTGCGTACCACACCCGTCGCATTGGAGAAGGGC ACGTAGAAGTTAGGGCCTTCTGTGCCATTCATGGCTGTGGCCCTTGTGG CTGACCC |
| AS300 | GGTGAAGCCACCTAGGACCATGAAGAGGTCAGCCACGGCTAGGTTGAG CAGGATGTAGTTGAGAGGCGTGCGCAGCTTCTTGTGCTGGACGGTGACG TAGAGCGTGAGGAAGTTGATGGGGAAGCCCAGCACGATCAGCAGAAAC ATGTAGGCGGCCAGCATGGAGAACTGCCATGGCTCAGCCAGGTAGTAC TGTGGGTACTCGAAGTGGCTGCGTACCACACCCGTCGCATTGGAGAAGG GCACGTAGAAGTTAGGGCCTTCTGTGCCATTCATGGCTGTGGCCCTTGT GGCTGACCC |

As a result, as can be seen in FIG. 10 and Table 2 below, in the case of AS50, AS100, and AS150, the self-circularization efficiency was relatively excellent compared to the length of AS200 or longer. On the other hand, for AS50 and AS100, total RNA produced after the in vitro transcription reaction was significantly less. Accordingly, it was found that AS 150 was the most optimal length for self-circularization RNA construct expression and circular RNA production.

**[Table 2]**

| AS series | AS50 | AS100 | AS150 | AS200 | AS250 | AS300 |
|---|---|---|---|---|---|---|
| A: Amount of Total RNA produced (µg) | 69.5 ± 1.77 | 9.15 ± 0.46 | 176.5 ± 3.89 | 140 ± 3.54 | 133 ± 2.83 | 141 ± 5.66 |
| B: Relative band intensity of circular RNA (%) | 6.35 ± 0.25 | 6.75 ± 0.04 | 5.95 ± 0.18 | 4.30 ± 0.07 | 4.20 ± 0.07 | 3.60 ± 0.14 |
| Relative factor (A * B) | 441 | 62 | 1050 | 602 | 559 | 508 |

### Example 6. Spacer Region Optimization

Subsequently, in order to identify the effect of the length or type of the spacer region on the immediate circularization reaction during an in vitro transcription process, a DNA template including spacer regions of different lengths and nucleotide sequences was produced (FIG. 11). A vector capable of expressing each RNA construct was produced in a manner like the one used for Example 1, and in vitro transcription was performed at 37°C for 3 hours in a manner like the one used for Example 2. The degree of immediate STS reaction was compared and identified through relative band intensity by performing PAGE on 4% polyacrylamide-7 M urea gel (20×20 cm, 1 mm). The nucleotide sequences of each spacer region are shown in Table 3 below. In this experiment, the spacers of A10, A30, and A50 were used by adding a restriction site at the 3' end for IRES insertion immediately after the spacer region. In this experiment, the AatII site (GACGTC) was added to the 3' end of the spacer and used.

**[Table 3]**

| Sections | Nucleotide sequences (5' --> 3') |
|---|---|
| A10 | AAAAAAAAAA |
| A30 | AAAAAAAAAAAAAAAAAAAAAAAAAAAAAA |
| A50 | AAAAAAAAAAAAAAAAAAAAAAAAAAAAAAAAAAAAAAAA AAAAAAAAAA |
| Control spacer 1 | GGTAGTGGTGCTACTAACTTCAGCCTGCTGAAGCA |
| Control spacer 2 | GGTAGTAAACTACTAACTACAACCTGCTGAAGCA |

As a result, as can be seen in FIG. 12 and Table 4 below, despite the difference in the length and sequence of the spacer, it was found that there was no significant difference in the immediate circularization efficiency during the in vitro transcription process, but that A30 operated as the most optimal spacer for self-circularization RNA construct expression and circular RNA production.

**[Table 4]**

| AS150 | A10 | Control spacer 1 | Control spacer 1 | A30 | A50 |
|---|---|---|---|---|---|
| Spacer version | | | | | |
| A: Amount of Total RNA produced (µg) | 199.5 ± 9.19 | 199.5 ± 6.36 | 187 ± 5.66 | 190 ± 8.49 | 192.5 ± 0.71 |
| B: Relative band intensity of circular RNA (%) | 5.55 ± 0.35 | 6.7 ± 0.14 | 6.15 ± 0.07 | 6.5 ± 1.27 | 5.7 ± 0.85 |
| Relative factor (A * B) | 1106 ± 19 | 1311 ± 70 | 1150 ± 21 | 1240 ± 297 | 1097 ± 160 |

### Example 7. Optimization of Self-circularization RNA Construct

### 7-1. AS Region, and P1 Helix and P10 Helix Regions

The self-circularization RNA construct designed in Example 1 includes an AS region, and P1 helix and P10 helix regions. In order to identify the effect of each configuration on the immediate circularization reaction during the in vitro transcription process, as illustrated in FIG. 13, a DNA template including only the P1 helix region, only the P1 and P10 helix regions, or both the P1 and P10 helixes and the AS region was prepared. A vector capable of expressing each RNA construct was produced in a manner like the one used for Example 1, and in vitro transcription was performed at 37°C for 3 hours in a manner like the one used for Example 2. The degree of immediate STS reaction was compared and identified through relative band intensity by performing PAGE on 4% polyacrylamide-7 M urea gel (20×20 cm, 1 mm).

The nucleotide sequences of the T7 DNA template including only the P1 helix region are shown in FIG. 14.

As a result, as can be seen in FIG. 15 and Table 5 below, it was found that there was no significant difference in the total RNA transcribed and generated in vitro for each vector, but that the amount of circular RNA produced was higher in the order of the case where both the P1 and P10 helixes and the AS region were included, the case where only the P1 helix region was included, and the case where only the P1 and P10 regions were included.

**[Table 5]**

| AS150 | P1 (No AS) | P1&P10 (No AS) | P1&P10 (AS150) |
|---|---|---|---|
| A: Amount of Total RNA produced (µg) | 200 | 196 | 182 |
| B: Relative band intensity of circular RNA(%) | 3.2 | 0.5 | 5.1 |
| Relative factor (A * B) | 640 | 98 | 928 |

7-2. Circularization Verification of Self-circularization RNA Construct that Does Not Include P10 and AS Regions

From the results of Example 7-1, it was found that sufficient circRNAs could be produced without an additional circularization stage during the in vitro transcription process from a DNA template (P1 construct) that does not include P10 and AS regions. In order to verify this finding, the product after the STS reaction of Example 7-1 was treated with RNase R to remove linear RNA, PAGE was performed in a manner like the one used for the previous experiment, and then RT-PCR and sequencing were performed in a manner like the one used for Example 2-2.

As a result, as can be seen in FIGS. 16A and 16B, even when self-circularization was performed using the P1 construct, upon treatment with RNase R, an RNA band that was not easily cut by RNase R and enriched was observed. As a result of performing RT-PCR and sequencing on the STS reaction product in the band, it was identified that the product was a circRNA.

### Example 8. Optimization of P1 Helix Region

### 8-1. P1 Helix Region and Self-Circularization

From the results of Example 7, it was identified that the self-circularization construct without P10, AS, and ABS is sufficient to make a circRNA. Accordingly, the intention was to verify assuming that a circular RNA would be generated if only the Internal Guide Sequence (IGS) present at the 5' end and the nucleotide sequence of the target site present at the 3' end constituting the P1 helix are complementarily bound to each other (U and G are wobble base pairs).

Since the IGS region is GNNNNN and the nucleotide sequence of the target site is N'N'N'N'N'U, when only one U nucleotide is added to the GOI, the finally generated circRNA consists only of one additional U base and the GOI region (FIGS. 1A and 1B). In addition, when the 3' end of the GOI ends with a U base, by designing the nucleotide sequence of the IGS region to be reverse-complementary to the GOI, the circRNA to be finally generated may be prepared such that it consists only of the GOI region (FIGS. 20A and 20B).

As shown in FIG. 17, various sequences of IGS and target site were designed, a vector was produced by the method of Example 1, and then the in vitro transcription (IVT) of Example 2-1 was performed.

As a result, as can be seen in FIGS. 18-19 and Table 6 below, it was identified that when both ends have complementary sequences, there is an increase in the amount of circRNA generated through the immediate STS reaction after in vitro transcription. In particular, it was identified that the efficiency of circRNA production was higher when the sequences of IGS and target site were AU-rich sequences. On the other hand, it was found that the vector including the no-complement IGS region generated a very low amount of circRNA. From the above, it was found that both the IGS and the target site can efficiently induce a self-circularization reaction when they are complementary to each other.

**[Table 6]**

| P1 variants (No AS) | AS150 (P1&P10) | RZ004 | RZ001 | RZ003 | GC-rich | AU-rich | 2 sites | No complement |
|---|---|---|---|---|---|---|---|---|
| Relative band intensity of circular RNA(%) | 5.4 | 3.7 | 7.9 | 6.6 | 5.4 | **11.6** | 6.1 | 1 |

### 8-2. Optimization of P1 Helix Region

It was found that when the sequence of the IGS and the sequence of the target site are complementary, an immediate STS reaction occurs after in vitro transcription, but that the STS reaction occurs more actively, especially in the sequences of the Au-rich IGS and the target site. Subsequently, in order to determine whether a specific sequence among the bases of A and U constituting the P1 helix affects the efficiency of self-circularization, a total of 32 combinations of AU-rich IGS were designed, and a target site was constructed to have a sequence complementary to that of the IGS. Then, an experiment was performed in a manner like the one used for Example 8-1 (FIG. 21). To set the same conditions, a GOI was selected such that there were no 32 AU-rich sequences. In order to determine whether the IGS could complementarily bind to regions other than the target site, the AU-rich sequences complementary to the IGS in the produced self-circularization RNA constructs were counted. One sequence complementary to IGSs 17 and 18 was identified within the ribozyme sequence, and no sequence complementary to the other IGSs was identified within the above mentioned RNA constructs. On the other hand, since a ribozyme may not be used to target and splice its internal sequence, the circular RNA produced from the self-circularization RNA construct expression vector containing IGSs 17 and 18 was generated by targeting and splicing by ribozyme through complementary binding between the IGS and the target site.

A 20 uL scale (1 ug T7 DNA template, 1 X Reaction buffer, 10 mM each ATP, UTP, CTP, GTP, T7 RNA polymerase mix 2 ul) was reacted for 3 hours at 37°C, then 29 uL of nuclease-free water was added. Afterwards, 1 uL of RNase-free DNase I (10 U/ul) was added thereto and then the mixture was reacted at 37°C for 30 minutes. Subsequently, column purification was performed using a Monarch RNA cleanup kit (NEB), and the concentration was measured using Nanodrop (Thermo Fisher Scientific product) equipment. 250 ng of each sample was mixed well with 10 M Urea-BPB (1X TBE) dye with a minimum 1: 1 sample:dye ratio (or higher dye ratio), heated at 75°C for 5 minutes, and then electrophoresed on 4% Polyacrylamide-7 M Urea denaturing PAGE (SYBR Gold Nucleic Acid Stain, Thermo Fisher Scientific) at 50°C and under 50 W conditions for 2 hours. As a result, it was confirmed that a circular RNA was produced.

The results under IGS conditions 1 to 16 are as shown in FIG. 22A, and the results show that relative band intensity was high under IGS conditions 14, 15, and 16. Among the IGS conditions it was found that the self-circularization efficiency was highest under IGS sequence 16.

For remaining IGS conditions 17 to 32, the results are shown in FIG. 22B, and the results show that relative band intensity was high under IGS sequences 17, 18, 25, and 28 to 32. Considering the intensity and consistency of the circular RNA band, through performing experiments repeatedly, it was found that the AU-rich IGS sequence consisting of two A bases and three U bases had a relatively high self-circularization efficiency, and that the self-circularization efficiency was highest in IGS sequence 16, and next highest in IGS sequence 28.

### Example 9. GOI Reconstruction

Additionally, in order to construct a circRNA consisting of only the GOI, the present inventors selected a GOI region capable of forming wobble base pairs with IGS as a target site to form a circRNA. However, when a target site is selected within the GOI to form circRNA, the GOI in the downstream region of the target site will be cleaved by the ribozyme and not included in the circRNA. Accordingly, it is necessary to reconstruct the GOI located upstream and downstream of the target site region (FIG. 23A). Hereinafter, the GOI region located downstream of the target site region is referred to by the "3' Region of GOI", and the GOI region other than the 3' Region of GOI is referred to by the "5' Region of GOI".

The present inventors designed the formed circRNAs such that they consist of an intact GOI by selecting five bases located upstream of the U base (included) capable of forming wobble base pairs within the GOI as the target site, and reconstructing the GOI such that the 3' region GOI located downstream of the U base is located upstream of the 5' region GOI.

Meanwhile, the GOI may contain two or more U bases capable of forming wobble base pairs. Therefore, if multiple candidate regions of the target site exist within the GOI, for circRNA production efficiency, the target site was selected such that the five consecutive bases upstream of the U base have a high content of A and U, as identified through the experiments of Example 8-2. In the drawings, regions with high content of A and U are labeled "AU rich".

FIG. 23B is a diagram illustrating an example of target site selection within a GOI and subsequent GOI reconstruction.

In the following, the present inventors attempted to identify whether the intended circRNA could be produced through the above-described target site selection and GOI reconstruction. Specifically, a target site was selected within the GOI using CVB3 as an IRES and using a sGFP, RLuc M185V/Q253A, or FLuc gene as a transgene, and the GOI was reconstructed to produce a circular RNA precursor expression vector, and then IVT was performed. For comparison, a circular RNA precursor was produced with a target site set in the spacer region. Herein, a target site located in a spacer region is referred to by "spacer target". The specific circular RNA precursors are illustrated in the drawings. Circular RNA production and yield thereof were identified by PAGE and HPLC using the IVT results of each circular RNA precursor.

### 9-1. GOI : CVB3 IRES-sGFP

The gene of interest was designed such that sGFP could be expressed by CVB3 IRES. In CVB3 IRES, two candidate regions for AU rich target sites were selected (denoted by AU11 and AU19, respectively). FIG. 23C shows the sequence of the gene of interest and two AU rich target site regions present therein. A spacer target was used as a control. The initially designed spacer region (AC40 spacer) does not have a region that could be a candidate for the target site. Meanwhile, previous studies have confirmed that the length of the spacer region does not affect circularization efficiency. Accordingly, the spacer target was manufactured by separately designing a AC108 spacer to be included in the circular RNA precursor.

The GOI located upstream and downstream of the AU11 or AU19 target site of the above mentioned gene of interest was reconstructed, and IVT was performed to confirm circRNA formation. FIG. 23D shows each region as a DNA template for expression of a circular RNA precursor when AU11 is selected as the target site. In the case where AU19 was selected as the target site, the GOI was reconstructed to design DNA templates, similar to the case where AU11 was selected as the target site. Then, each was prepared as a vector according to the method of Example 1, and in vitro transcription (IVT) of Example 2-1 was performed.

After IVT, PAGE was performed to confirm circRNA formation (FIG. 23E). As a result, it was confirmed that circular RNA precursors with AU11 and AU19 as target sites formed circRNA through the STS reaction, and that the efficiency of circularization was higher than that of the circular RNA precursors with the AU11 target site.

The above mentioned PAGE results were re-confirmed by performing IP-RP HPLC (FIGS. 24A to 24D). When self-circularization was performed using the AU11 target site and the sample treated with RNase R was analyzed, a high circular RNA peak was observed. On the other hand, the self-circularization construct using the AU19 target site, which has relatively low self-circularization efficiency, was found to have a relatively small circRNA peak when analyzed by HPLC after RNase R treatment, in the same manner as shown by the PAGE results. A 4% denaturing PAGE was performed on each peak (peaks in FIGS. 24B and 24D) identified from the above mentioned IP-RP HPLC results, and it was confirmed that a circular RNA was purified (FIG. 25).

Table 7 below shows the self-circularization efficiency and final yield of circular RNA precursors designed according to the selected target site within the GOI. AU16 and AU28 shown in Table 7 are not shown in FIG. 23C. The target site (5'-ACGGCU-3') located in the spacer in Table 7 is a control included for comparison.

**[Table 7]**

| IRES | P1 target site | Gene | Final yield of circular obtained (%) = the amount of circular RNA purified by IP-RP HPLC/the amount of IVT |
|---|---|---|---|
| CVB3 | AU16 | GFP | 6.2 |
| | AU28 | | 6.5 |
| | AU11 | | 22.9 |
| | AU19 | | 1.9 |
| | spacer | | 6.6 |

The above shown results suggest that the choice of AU rich target sites for a completely identical GOI sequence or P1 construct makes a significant difference in self-circularization efficiency and final yield.

### 9-2. GOI : CVB3 IRES-R.Luciferase (M185V/Q235A)

A gene of interest was designed so that the RLuc mutant (M185V/Q235A) could be expressed by the CVB3 IRES. The AU11 target site was selected within the sequences of the gene of interest, the GOI was reconstructed based on this selection to design DNA templates, each of which was prepared as a vector by the method of Example 1, and then in vitro transcription (IVT) of Example 2-1 was performed. A spacer target was used as a control. As in the experiments of Example 9-1, the circular RNA precursor used as a control was designed and constructed such that it includes an AC108 spacer.

FIG. 26A shows the sequence of the gene of interest and AU rich target site region present therein, and FIG. 26B shows the specific sequence and each region of the circular RNA precursor expression DNA template reconstructed with a GOI based on the selected AU11 target site region.

The results of PAGE performed after IVT are shown in FIG. 26C. The circular RNA precursor with AU11 as target site formed a circRNA through an STS reaction, which was re-confirmed by performing IP-RP HPLC. Purification of circular RNA was identified by performing a 4% denaturing PAGE on the HPLC peaks (FIG. 26D).

When an AU11 is used as the target site, the circular RNA yield is as shown in Table 8 below.

**[Table 8]**

| IRES | P1 target site | Gene | Final yield of circular obtained (%) = the amount of circular RNA purified/the amount of IVT |
|---|---|---|---|
| CVB3 | spacer | R.Luci | 2.1 |
| | AU11 | | 13.7 |

### 9-3. GOI : CVB3 IRES-F.Luciferase

Experiments were performed that were like the ones of Example 9-2 except that the transgene was changed to F.luciferase.

FIG. 27A shows the sequence of the gene of interest and AU rich target site region and spacer target site region present therein, and FIG. 27B shows the specific sequence and each region of the circular RNA precursor expression DNA template reconstructed with GOI based on the selected AU11 target site region. A spacer target was used as a control, and all circular RNA precursors in these experiments were designed and manufactured such that they include an AC108 spacer.

The results of PAGE performed after IVT are shown in FIG. 27C. The circular RNA precursor with AU11 as target site formed a circRNA through an STS reaction, which was re-confirmed by performing IP-RP HPLC. Purification of circular RNA was identified by performing a 4% denaturing PAGE on the HPLC peaks (FIG. 27D).

**[Table 9]**

| IRES | P1 target site | Gene | Final yield of circular obtained (%) = the amount of circular RNA purified/the amount of IVT |
|---|---|---|---|
| CVB3 | spacer | FLuc | N.C. |
| | AU11 (AC 108) | | 5.7 |

From the above shown results, it can be seen that regardless of the type of transgene within the gene of interest, a circular RNA containing only the intact form of the GOI is formed by the circular RNA precursor, even when the selected target site is within the gene of interest and the GOI is reconstructed accordingly.

Although a number of embodiments have been described with reference to limited drawings, one of ordinary skill in the art will recognize that various modifications and alterations may be made to these embodiments based on the above detailed description. For example, suitable results may be achieved if the described techniques are performed in a different order, and/or if components in a described system, architecture, device, or circuit are combined in a different manner, and/or replaced or supplemented by other components or their equivalents.

Therefore, other implementations, other examples, and equivalents to the claims are also within the scope of the following claims.

### [Industrial Applicability]

The present disclosure may be used to produce circRNAs for protein expression in vitro, in cells, and in vivo, and may be used to produce functional RNA such as miRNA, anti-miRNA, shRNA, aptamers, mRNA vaccines, mRNA therapeutic agents, antibodies, vaccine adjuvants, and CAR-T mRNA as a circRNA.

## Claims

1. A self-circularization RNA construct, wherein
the construct has a structure of 5' - IGS (internal guide sequence) - ribozyme - gene of interest - target site - 3',
the IGS region and the target site form a guanine (G) : uracil (U) wobble base pair,
the guanine forming the wobble base pair is located at the 5' end of the IGS region,
the uracil forming the wobble base pair is located at the 3' end of a target site region, and
the IGS region consists of adenine (A) or uracil in addition to the bases forming the wobble base pair.

2. The self-circularization RNA construct of claim 1, wherein the target site region overlaps a gene of interest region.

3. The self-circularization RNA construct of claim 1, wherein a nucleotide sequence of the IGS region is reverse complementary to a nucleotide sequence of the target site region except for the guanine.

4. The self-circularization RNA construct of claim 1, wherein the ribozyme is a Group I intron ribozyme.

5. The self-circularization RNA construct of claim 1, wherein the ribozyme comprises a nucleotide sequence represented by SEQ ID NO: 6.

6. The self-circularization RNA construct of claim 1, wherein the construct comprises a nucleotide extending in the 5' direction of the IGS region, forming a P10 helix.

7. The self-circularization RNA construct of claim 1, wherein the construct comprises a nucleotide extending in the 5' direction of the IGS region and the 3' direction of the target site, forming a P1 helix.

8. The self-circularization RNA construct of claim 7, wherein the construct does not form a P10 helix.

9. The self-circularization RNA construct of claim 1 or 6, wherein the construct further comprises an antisense sequence (AS) region in the 5' direction of the IGS region and an antisense binding sequence (ABS) region capable of complementary binding to the AS region in the 3' direction of the target site.

10. The self-circularization RNA construct of claim 9, wherein a length of the AS region is 50-nt to 400-nt.

11. The self-circularization RNA construct of claim 1, wherein the gene of interest region comprises an internal ribosome entry site (IRES) region at the 5' end.

12. The self-circularization RNA construct of claim 1, wherein the construct has a ribozyme region and a gene of interest region linked by a spacer region comprising a random nucleotide sequence.

13. The self-circularization RNA construct of claim 1, wherein the construct has a gene of interest region and a target site region linked by a spacer region comprising a random nucleotide sequence.

14. A vector expressing the self-circularization RNA construct of claim 1.

15. The vector of claim 14, wherein the vector comprises a promoter operably linked to a gene encoding the self-circularization RNA.

16. A self-circularization RNA construct, wherein
the construct has a structure of 5' - IGS (internal guide sequence) - ribozyme - reconstructed gene of interest - 3',
the reconstructed gene of interest is a gene of interest that has a different arrangement,
the gene of interest comprises a uracil (U) base,
the reconstructed gene of interest comprises a gene in which a 3' region of the gene of interest linked in the 3' direction of the uracil base is linked to a 5' end of the gene of interest, so that the uracil base is located at the 3' end of the gene of interest, and
the IGS region at its 5' end comprises a guanine (G) capable of forming a wobble base pair with a uracil base in the gene of interest, and comprises a sequence reverse complementary to a sequence of five consecutive bases linked in the 5' direction of the uracil base in the gene of interest.

17. The self-circularization RNA construct of claim 16, wherein the sequence of five consecutive bases linked in the 5' direction of the uracil base at the 3' end of the reconstructed gene of interest comprises from 1 to 5 of an adenine (A) and/or a uracil base.

18. The self-circularization RNA construct of claim 16, wherein the ribozyme is a Group I intron ribozyme.

19. The self-circularization RNA construct of claim 16, wherein the ribozyme comprises a nucleotide sequence represented by SEQ ID NO: 6.

20. The self-circularization RNA construct of claim 16, wherein the construct comprises a nucleotide extending in the 5' direction of the IGS region, and the extended nucleotide comprises a sequence reverse complementary to the 5' end of the reconstructed gene of interest, forming a P10 helix.

21. The self-circularization RNA construct of claim 16, wherein the construct comprises a nucleotide extending in the 5' direction of the IGS region and a nucleotide extending in the 3' direction of the reconstructed gene of interest, wherein each of the extended nucleotides is sequence reverse complementary to each other.

22. The self-circularization RNA construct of claim 16 or 20, wherein the construct further comprises an antisense sequence (AS) region in a 5' direction of the IGS region and an antisense binding sequence (ABS) region capable of complementary binding to the AS region in a 3' direction of the reconstructed gene of interest.

23. The self-circularization RNA construct of claim 22, wherein a length of the AS region is 50-nt to 400-nt.

24. The self-circularization RNA construct of claim 16, wherein the construct has a ribozyme region and a reconstructed gene of interest region linked by a spacer region comprising a random nucleotide sequence.

25. A vector expressing the self-circularization RNA construct of claim 16.

26. The vector of claim 25, wherein the vector comprises a promoter operably linked to a gene encoding the self-circularization RNA.
